# EUROPEAN PATENT APPLICATION

(11) **EP 1 598 367 A1**
(43) Date of publication of application: **23.11.2005**
(21) Application number: 04011736.8
(22) Date of filing: 18.05.2004
(51) Int. Cl.: C07K 14/745, C07K 14/755, C12N 15/12

(54) **Modified coagulation factors with enhanced stability and their derivatives**

(71) Applicant: ZLB Behring GmbH, 35041 Marburg (DE)
(72) Inventor: Weimer, Thomas, Dr., 35075 Gladenbach (DE); Schulte, Stefan, Dr., 35043 Marburg (DE); Hofmann, Kay, Dr., 50823 Köln (DE); Hauser, Hans-Peter, Dr., 35041 Marburg (DE)

(57) **Abstract**

The present invention relates to modified nucleic acid sequences coding for coagulation Factors, in particular human FV and human Factor VIII and their derivatives with improved stability, recombinant expression vectors containing such nucleic acid sequences, host cells transformed with such recombinant expression vectors, recombinant polypeptides and derivatives which do have biological activities of the unmodified wild type protein but having improved stability and processes for the manufacture of such recombinant proteins and their derivatives. The invention also covers a transfer vector for use in human gene therapy, which comprises modified DNA sequences.

## Description

### Field of the invention:

The present invention relates to modified nucleic acid sequences coding for coagulation Factors, in particular human FV and human Factor VIII and their derivatives with improved stability, recombinant expression vectors containing such nucleic acid sequences, host cells transformed with such recombinant expression vectors, recombinant polypeptides and derivatives which do have biological activities of the unmodified wild type protein but having improved stability and processes for the manufacture of such recombinant proteins and their derivatives. The invention also relates to a transfer vector for use in human gene therapy, which comprises such modified nucleic acid sequences.

### Background of the invention:

Classic hemophilia or hemophilia A is an inherited bleeding disorder. It results from a chromosome X-linked deficiency of blood coagulation Factor VIII, and affects almost exclusively males with an incidence of between one and two individuals per 10.000. The X-chromosome defect is transmitted by female carriers who are not themselves hemophiliacs. The clinical manifestation of hemophilia A is an increased bleeding tendency. Before treatment with Factor VIII concentrates was introduced the mean life span for a person with severe hemophilia was less than 20 years. The use of concentrates of Factor VIII from plasma has considerably improved the situation for the hemophilia patients increasing the mean life span extensively, giving most of them the possibility to live a more or less normal life. However, there have been certain problems with the plasma derived concentrates and their use, the most serious of which have been the transmission of viruses. So far, viruses causing AIDS, hepatitis B, and non-A non-B hepatitis have hit the population seriously. Since then different virus inactivation methods and new highly purified Factor VIII concentrates have recently been developed which established a very high safety standard also for plasma derived Factor VIII.

The cloning of the cDNA for Factor VIII (Wood, W.I., et al. (1984) Nature 312, 330-336; Vehar, G.A., et al. (1984) Nature 312, 337-342) made it possible to express Factor VIII recombinantly leading to the development of several recombinant Factor VIII products, which were approved by the regulatory authorities between 1992 and 2003. The fact that the central B domain of the Factor VIII polypeptide chain residing between amino acids Arg-740 and Glu-1649 does not seem to be necessary for full biological activity has also led to the development of a B domain deleted Factor VIII.

The mature Factor VIII molecule consists of 2332 amino acids which can be grouped into three homologous A domains, two homologous C domains and a B Domain which are arranged in the order: A1-A2-B-A3-C1-C2. The complete amino acid sequence of mature human Factor VIII is shown in SEQ ID NO:2. During its secretion into plasma Factor VIII is processed intracellularly into a series of metal-ion linked heterodimers as single chain Factor VIII is cleaved at the B-A3 boundary and at different sites within the B-domain. This processing leads to a heavy chain consisting of the A1, the A2 and various parts of the B-domain which has a molecular size ranging from 90 kDa to 200 kDa. The heavy chains are bound via a metal ion to the light chain, which consists of the A3, the C1 and the C2 domain (Saenko et al. 2002). In plasma this heterodimeric Factor VIII binds with high affinity to von Willebrand Factor, which protects it from premature catabolism. The half-life of non-activated Factor VIII bound to VWF is about 12 hours in plasma.

During coagulation Factor VIII is activated via proteolytic cleavage by FXa and thrombin at amino acids Arg372 and Arg740 within the heavy chain and at Arg1689 in the light chain resulting in the release of von Willebrand Factor and generating the activated Factor VIII heterotrimer which will form the tenase complex on phospholipid surfaces with FIXa and FX provided that Ca²⁺ is present. The heterotrimer consists of the A1 domain, a 50 kDa fragment, the A2 domain a 43 kDa fragment and the light chain (A3-C1-C2), a 73 kDa fragment. Thus the active form of Factor VIII (Factor VIIIa) consists of an A1-subunit associated through the divalent metal ion linkage to a thrombin-cleaved A3-C1-C2 light chain and a free A2 subunit relatively loosely associated with the A1 and the A3 domain.

To avoid excessive coagulation Factor VIIIa must be inactivated soon after activation. The inactivation of Factor VIIIa via activated Protein C (APC) by cleavage at Arg336 and Arg562 is not considered to be the rate-limiting step. It is rather the dissociation of the non covalently attached A2 subunit from the heterotrimer which is thought to be the rate limiting step in Factor VIIIa inactivation after thrombin activation (Fay, P.J. et al, J. Biol. Chem. 266: 8957 (1991), Fay PJ & Smudzin TM, J. Biol. Chem. 267: 13246-50 (1992)). This is a rapid process, which explains the short half-life of Factor VIIIa in plasma which is only 2.1 minutes (Saenko et al., Vox Sang. 83: 89-96 (2002)). Therefore increasing the affinity of the A2 domain to the A1/A3-C1-C2 heterodimer would prolong the half-life of Factor VIIIa and a Factor VIII with increased haemostatic activity would be obtained.

In severe hemophilia A patients undergoing prophylactic treatment Factor VIII has to be administered i.v. about 3 times per week due to the short plasma half life of Factor VIII of about 12 hours. Each i.v. administration is cumbersome, associated with pain and entails the risk of an infection especially as this is mostly done in home treatment by the patients themselves or by the parents of children being diagnosed for hemophilia A.

It would thus be highly desirable to create a Factor VIII with increased functional half-life allowing the manufacturing of pharmaceutical compositions containing Factor VIII, which have to be administered less frequently.

Several attempts have been made to prolong the half-life of non-activated Factor VIII either by reducing its interaction with cellular receptors (WO 03/093313A2, WO 02/060951A2), by covalently attaching polymers to Factor VIII (WO 94/15625, WO 97/11957 and US 4970300) or by encapsulation of Factor VIII (WO 99/55306).

In WO 97/03193 it was speculated that the introduction of novel metal binding sites could stabilize Factor VIII and in particular mutants in which His or Met is substituted for any of Phe652, Tyr1786, Lys1818, Asp1840 and/or Asn1864. However no rationale was provided how to determine the success meaning the stabilization resulting from such modifications nor a rationale why the proposed amino acids were chosen.

Another approach has been made in creating a Factor VIIIa which is inactivation resistant by covalently attaching the A2 domain to the A3 domain and by mutating the APC cleavage sites (Pipe and Kaufman, PNAS, 94:11851-11856). This genetic construct was also used to produce transgenic animals as described in WO 02/072023A2.

Gale et al. (Protein Science 2002, 11:2091-2101) published the stabilization of FV by covalently attaching the A3 domain to the A2 domain. They identified two neighbouring amino acids according to structural predictions, one on the A2 domain and the other being located on the A3 domain, and replaced these two amino acids with cysteine residues which formed a disulfide bridge during export into the endoplasmatic reticulum. The same approach was used to covalently attach via disulfide bridges the A2 to the A3 domain of Factor VIII (WO 02/103024A2). Such covalently attached Factor VIII mutants retained about 90% of their initial highest activity for 40 minutes after activation whereas the activity of wild type Factor VIII quickly went down to 10% of its initial highest activity. The Factor VIII mutants retained their 90% activity for additional 3h without any further loss of activity (Gale et al., J. Thromb. Haemost., 2003, 1: p. 1966-1971).

It has however recently been shown that constitutively high levels of Factor VIII might constitute a risk factor for thromboembolism (Kyrle 2003, Hämostasiologie 1: p. 41-57). A Factor VIII with a covalent attachment of the A3 and the A2 domain therefore entails a potential for thrombotic events as it might be too resistant to inactivation so the life saving negative feedback mechanisms after hemostasis is achieved might be compromised.

Hence, there is an ongoing need to develop modified blood coagulation Factors which exhibit favourable properties.

In the present invention it has been surprisingly found that after a careful sequence analysis of Factor VIII combined with modelling the structure of Factor VIII based on the corresponding 3-D structure of ceruloplasmin a clear rationale for the introduction of novel metal binding sites within Factor VIII could be developed. Introducing the proposed mutations leads to the stabilization of Factor VIII and/or an increase of its functional half-life without the drawback of a stabilization by covalent attachment which might result into a Factor VIIIa which is too stable and potentially thrombogenic.

In a first aspect, the invention therefore relates to a method of producing a modified homologue of human ceruloplasmin, comprising adding at least one metal binding site to a homologue of human ceruloplasmin, wherein the metal binding site corresponds to a metal binding site in human ceruloplasmin. The invention further relates to a method of producing a modified homologue of human ceruloplasmin, comprising adding at least one metal binding site to a homologue of human ceruloplasmin, wherein the metal binding site corresponds to an incomplete metal binding site in human ceruloplasmin.

Preferably, the method comprises the following steps:
a) identifying the amino acids in the amino acid sequence of the homologue of human ceruloplasmin, which correspond to the amino acids in the amino acid sequence of human ceruloplasmin forming a metal binding site in human ceruloplasmin, or forming an incomplete metal binding site in human ceruloplasmin wherein the identified amino acids in the amino acid sequence of the homologue of human ceruloplasmin do not form a metal binding site;
b) identifying one or more amino acid mutations which would add such metal binding site to the homologue of human ceruloplasmin;
c) preparing a polynucleotide encoding a modified homologue of human ceruloplasmin, wherein the modified homologue comprises at least one metal binding site according to (b); and
d) expressing the polynucleotide in a host cell and recovering the modified homologue.

### Step a) may comprise the following substeps:

(aa) optionally, identifying in the three-dimensional structure of human ceruloplasmin a metal ion bound by human ceruloplasmin;
(bb) identifying the amino acids in the amino acid sequence of human ceruloplasmin forming a metal binding site in human ceruloplasmin, or forming an incomplete metal binding site in human ceruloplasmin;
(cc) identifying the amino acids in the amino acid sequence of the homologue of human ceruloplasmin, which correspond to the amino acids in the amino acid sequence of human ceruloplasmin identified in substep (bb);
(dd) selecting the amino acids identified in substep (cc) if they are not capable of forming a metal binding site.

Human ceruloplasmin is an oxidoreductase present in human plasma. The amino acid sequence of human ceruloplasmin is shown in SEQ ID NO:5. The three-dimensional structure of human ceruloplasmin has been solved by X-ray crystallography (Zaitseva et al., 1996, J Biol Inorg Chem 1, pp 15 et seq.). The structural data are available from various data bases under PDB file number 1 KCW.

As used herein, the term "homologue of human ceruloplasmin" denotes a polypeptide comprising
(1) an amino acid sequence having an identity of at least 30 % to the A1 domain of human ceruloplasmin (amino acids 2 to 338 of the amino acid sequence as shown in SEQ ID NO:5);
(2) an amino acid sequence having an identity of at least 30 % to the A2 domain of human ceruloplasmin (amino acids 352 to 699 of the amino acid sequence as shown in SEQ ID NO:5); and/or
(3) an amino acid sequence having an identity of at least 30 % to the A3 domain of human ceruloplasmin (amino acids 712 to 1040 of the amino acid sequence as shown in SEQ ID NO:5).

The degree of identity between two amino acid sequences may be determined using methods known to those skilled in the art. Preferably, the degree of identity between two amino acid sequences is determined using the program "lalign", which is a part of the "FASTA V3.0" program package of William Pearson. Other programs based on the 'Smith-Waterman' algorithm may also be employed. The parameter settings are as follows:
- substitution matrix BLOSUM45;
- 'gap creation penalty' 2.0, and
- 'gap extension penalty' 0.2

Preferred homologues of human ceruloplasmin according to this invention include blood coagulation Factor V and blood coagulation Factor VIII. Even more preferred are human blood coagulation Factor V and human blood coagulation Factor VIII.

The terms "blood coagulation Factor VIII", "Factor VIII" and F VIII" are used interchangeably herein. "Blood coagulation Factor VIII" includes derivatives of wild type blood coagulation Factor VIII having the proagulant activity of wild type blood coagulation Factor VIII. Derivatives may have deletions, insertions and/or additions compared with the amino acid sequence of wild type Factor VIII. As non-limiting examples, Factor VIII molecules include full length recombinant Factor VIII, B domain deleted Factor VIII (Pittman 1993, Blood 81:2925-2935), Factor VIII mutants preventing or reducing APC cleavage (Amano 1998, Thromb. Haemost. 79:557-563), Factor VIII mutants further stabilizing the A2 domain (WO 97/40145), FVIII mutants resulting in increased expression (Swaroop et al. 1997, JBC 272:24121-24124), Factor VIII mutants reducing its immunogenicity (Lollar 1999 Thromb. Haemost. 82:505-508), FVIII reconstituted from differently expressed heavy and light chains (Oh et al. 1999, Exp. Mol. Med. 31:95-100), FVIII mutants reducing binding to receptors leading to catabolism of FVIII like HSPG (heparan sulfate proteoglycans) and/or LRP (low density lipoprotein receptor related protein) (Ananyeva et al. 2001, TCM, 11:251-257. A suitable test to determine the procoagulant activity of Factor VIII is the one stage or the two stage coagulation assay (Rizza et al. 1982 Coagulation assay of FVIIIc and FIXa in Bloom ed. The Hemophilias. NY Churchchill Livingston 1992)

The terms "blood coagulation Factor V", "Factor V" and F V" are used interchangeably herein. "Blood coagulation Factor V" includes derivatives of wild type blood coagulation Factor V having the proagulant activity of wild type blood coagulation Factor V. Derivatives may have deletions, insertions and/or additions compared with the amino acid sequence of wild type Factor V. Non-limiting examples of Factor V molecules include full length recombinant Factor V, Factor V Leiden, Factor V Hong Kong, Factor V Cambridge (Marquette et al, Blood 86:3026pp, Thorelli 1997, Eur. J. Biochem. Nicolaes et al. 2003, Hematol. Oncol. North Am. 17:37-61). A suitable test to determine the procoagulant activity of Factor V is (Kane WH 1981, JBC, 256:1002).

Also chimeric molecules of FV and FVIII are one aspect of the invention (Marquette et al. 1995, JBC, 270:10297-10303, Oertel et al. 1996, Thromb. Haemost. 75:36-44).

The cDNA sequence and the amino acid sequence of the mature wild type form of human blood coagulation Factor VIII are shown in SEQ ID NO:1 and SEQ ID NO:2, respectively. The cDNA sequence and the amino acid sequence of the mature wild type form of human blood coagulation Factor V are shown in SEQ ID NO:3 and SEQ ID NO:4, respectively. The indication of amino acid positions herein allows the identification of the respective amino acid residues within the sequence of a polypeptide, e.g. in the homologue of human ceruloplasmin. The reference to an amino acid position of a specific sequence does not exclude the presence of mutations, e.g. deletions, insertions and/or substitutions at other positions in the sequence referred to. For example, a mutation in "Glu2004" referring to SEQ ID NO:2 does not exclude that in the modified homologue one or more amino acids at positions 1 through 2003 of SEQ ID NO:2 are missing.

In a first step, a metal binding site in human ceruloplasmin may be identified using the 3D structure of human ceruloplasmin. The amino acids in SEQ ID NO:5 coordinating the metal atom can be identified in that way. Generally, there are two major types of metal binding sites in human ceruloplasmin. In the first type, all amino acids coordinating the metal atom are comprised in one domain (A1, A2 or A3 domain). This first type of metal binding site is termed "intra-subunit binding site" herein. The preferred amino acids forming an intra-subunit binding site are His (position 1), Cys (position 2), His (position 3) and Met (position 4). In the second type, amino acids from two different domains of human ceruloplasmin form the metal binding site in human ceruloplasmin. This second type of metal binding site is termed "inter-subunit binding site" herein. The preferred amino acids forming an inter-subunit binding site are Glu (position 1), His (position 2), Asp (position 3) and Glu (position 3).

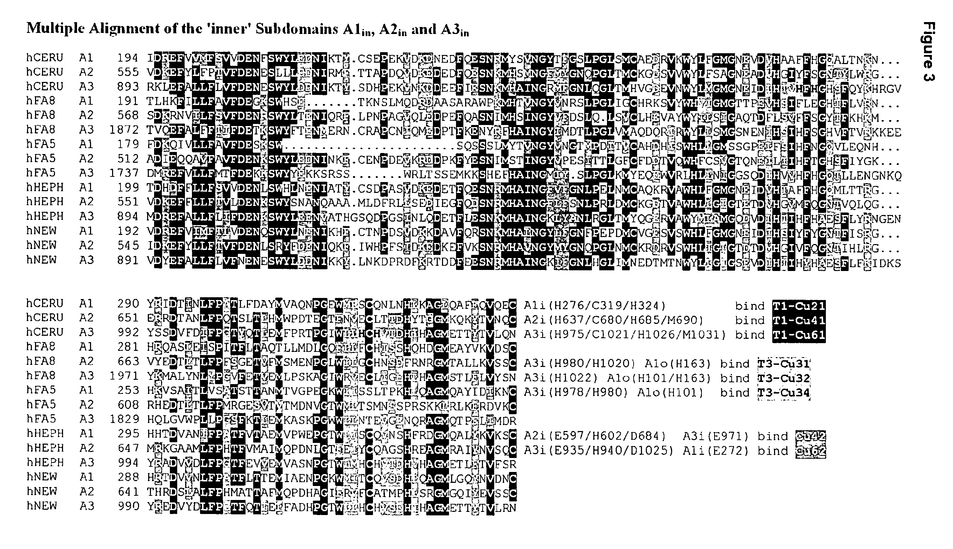

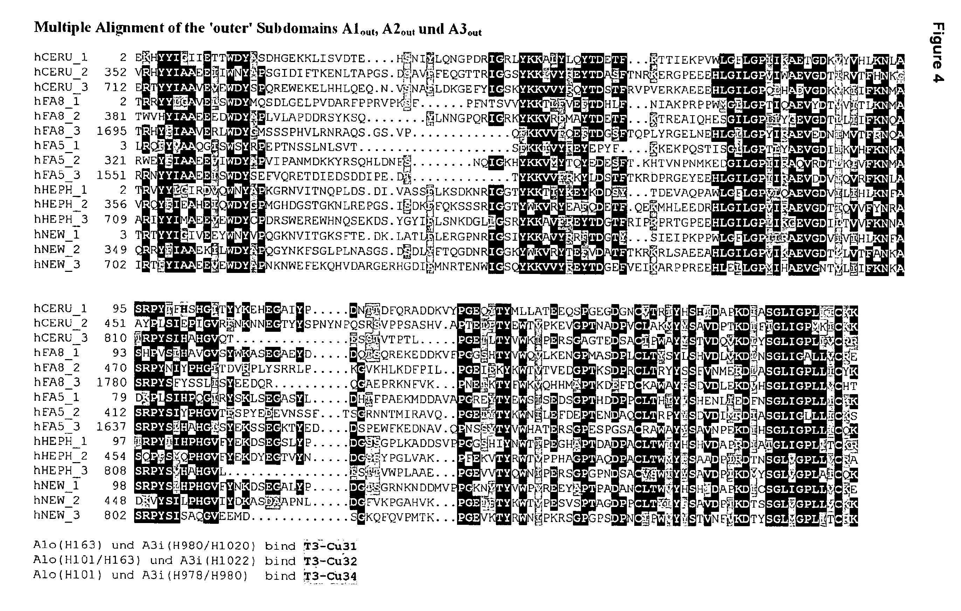

As shown in Figures 3 and 4, the A1, A2 and A3 domains of human ceruloplasmin exhibit a rather high degree of sequence identity. Due to this structural similarity of the A1, A2 and A3 domain of human ceruloplasmin, the information on a metal binding site found in a first domain can be applied to the second and/or third domain. As used herein, an "incomplete binding site" refers to a group of amino acids which do not form a metal binding site but which can be identified by analogy to a metal binding site of one or more other subdomains in the same molecule. Therefore also incomplete metal binding sites of human ceruloplasmin can be identified and completed by analogy to binding sites of other subdomains, e.g. the incomplete copper binding site between the A1 and the A2 domain of ceruloplasmin consisting of Glu236, Tyr241, Asn323 and Glu272 can be replaced by copper coordinating amino acids from the homologous regions between the A2 and the A3 domain (Glu597, His602, Asp684 and Glu971) as well as with those between the A3 and the A1 domain (Glu935, His940, Asp1025 and Glu272).

By way of example, three metal binding sites (i) to (iii) in human ceruloplasmin have been identified, and the metal-coordinating amino acids are given in the following:
(i) His637, Cys680, His 685 and Met 690;
(ii) Glu597, His602, Asp684 and Glu971;
(iii) Glu935, His940, Asp1025 and Glu272;
   wherein the amino acid numbering refers to the amino acid sequence of human ceruloplasmin as shown in SEQ ID NO:5. Binding site (i) is an intra-subunit binding site, whereas binding sites (ii) and (iii) are inter-subunit binding sites. In addition, the amino acids forming an incomplete metal binding site (iv) in human ceruloplasmin have been identified:
(iv) Glu236, Tyr241, Asn323 and Glu633.

The order (sequence) of amino acids, referring to the positions in the binding site, in any one of the above lines (i) to (iv) is: position 1, position 2, position 3 and position 4.

By way of sequence comparison (alignment) and/or 3-D modelling, it can then be determined whether the homologue of human ceruloplasmin comprises a corresponding metal binding site. If sequence comparison (alignment) is employed, the above-mentioned method for determining the degree of identity between two amino acid sequences is used. If 3-D modelling is employed, the program "DeepView/Swiss-PDB Viewer", Version 3.7 can be used.

To accomplish this, the amino acids in the amino acid sequence of the homologue of human ceruloplasmin, which correspond to the amino acids in the amino acid sequence of human ceruloplasmin forming a metal binding site or an uncomplete metal binding site are identified.

Usually, an amino acid in the amino acid sequence of the homologue of human ceruloplasmin, which "corresponds" to an amino acid in the amino acid sequence of human ceruloplasmin, can be identified using the program for determining the degree of identity between two amino acid sequences and the parameters described above. If the sequence comparison results in a given amino acid aa1 of the homologue of human ceruloplasmin being aligned below an amino acid aa2 of human ceruloplasmin the amino acids aa1 and aa2 may be considered as "corresponding" to each other. For example, an amino acid of Factor VIII or Factor V "corresponding" to a given amino acid of human ceruloplasmin can also be identified with the help of the alignment as shown in Figures 3 and 4.

The amino acid positions in the amino acid sequence of human Factor VIII, corresponding to the amino acid positions forming the metal binding sites (i) to (iii) and the incomplete binding site (iv) above are the following:
(i) Leu649, Cys692, Phe697 and Met702;
(ii) Ala610, His615, Asp696 and Asn1950;
(iii) Glu1914, His1919, Glu2004 and Thr263;
(iv) Ala227, His232, Ser314 and Gln645;
wherein the amino acid numbering refers to the amino acid sequence of human blood coagulation Factor VIII as shown in SEQ ID NO:2.

Whether the corresponding amino acids of the homologue of human ceruloplasmin form a metal binding site may be determined by examining whether at least one of the corresponding amino acids is not a preferred amino acid at the respective position and not an amino acid homologous thereto. As indicated supra, the preferred amino acids in the case of an intra-subunit binding site are His (position 1), Cys (position 2), His (position 3) and Met (position 4). The preferred amino acids in the case of an inter-subunit binding site are Glu (position 1), His (position 2), Asp (position 3) and Glu (position 4). As to amino acids homologous to the preferred amino acids, the following rules can be applied in accordance with this invention: Glu is homologous to Asp, Asp is homologous to Glu, Cys is homologous to His, and His is homologous to Cys.

The amino acids Leu649 (position 1) and Phe697 (position 3) in the FVIII sequence are neither preferred amino acids nor amino acids homologous to the preferred amino acids at the respective positions of an intra-subunit binding site. Accordingly, the amino acids Leu649, Cys692, Phe697 and Met702 do not form a metal binding site in Factor VIII.

The amino acids Ala610 (position 1) and Asn1950 (position 4) in the FVIII sequence are neither preferred amino acids nor amino acids homologous to the preferred amino acids at the respective positions of an inter-subunit binding site. Accordingly, the amino acids Ala610, His615, Asp696 and Asn1950 do not form a metal binding site in Factor VIII.

The amino acid Thr263 (position 4) in the FVIII sequence is neither a preferred amino acid nor an amino acid homologous to the preferred amino acid at the respective position of an inter-subunit binding site. Accordingly, the amino acids Glu1914, His1919, Glu2004 and Thr263 do not form a metal binding site in Factor VIII.

The amino acids Ala227 (position 1), Ser314 (position 3) and Gln645 (position 4) in the FVIII sequence are neither preferred amino acids nor amino acids homologous to the preferred amino acids at the respective positions of an inter-subunit binding site. Accordingly, the amino acids Ala227, His232, Ser314 and Gln645 do not form a metal binding site in Factor VIII.

If at least one of the corresponding amino acids in the sequence of the homologue differs from the preferred amino acid of the respective type of metal binding site, the corresponding amino acid in the sequence of the homologue may be mutated.

Accordingly, the amino acids of at least one of the following groups (i) to (iv) of amino acids in the sequence of human blood coagulation Factor VIII may be substituted:
(i) Leu649 and Phe697;
(ii) Ala610 and Asn1950;
(iii) optionally Glu2004 and Thr263; and/or
(iv) Ala227, Ser314 and Gln645;
wherein the amino acid numbering refers to the amino acid sequence as shown in SEQ ID NO:2.

In one embodiment, only the amino acids of one of the above groups (i) to (iv) are substituted. Accordingly, the amino acids of group (i) or (ii) or (iii) or (iv) are substituted. In another embodiment, the amino acids of two or three or four of the above groups (i) to (iv) are substituted. This applies to other homologues of human ceruloplasmin *mutatis mutandis.*

A mutation may be a substitution, insertion and/or deletion. Preferably, the mutation is a substitution replacing an amino acid with a different amino acid. An amino acid in the sequence of the homologue of human ceruloplasmin identified as described above is preferably replaced with the preferred amino acid at that position, depending on the type of binding site (intra-subunit or inter-subunit). As indicated supra, the preferred amino acids in the case of an intra-subunit binding site are His (position 1), Cys (position 2), His (position 3) and Met (position 4). The preferred amino acids in the case of an inter-subunit binding site are Glu (position 1), His (position 2), Asp (position 3) and Glu (position 4).

Alternatively, the amino acid in the sequence of the homologue of human ceruloplasmin identified as described above may be replaced with an amino acid that is homologous to the preferred amino acid at that position. In accordance with this invention, Glu is homologous to Asp, Asp is homologous to Glu, Cys is homologous to His, and His is homologous to Cys. Replacement with homologous amino acids, however, is not preferred in this invention.

Preferably, one or more of the following groups (i) to (iv) of substitutions in Factor VIII are effected:
(i) Leu649→His and Phe697→His;
(ii) Ala610→Glu and Asn1950→Glu;
(iii) optionally Glu2004→Asp and Thr263→Glu; and/or
(iv) Ala227→Glu, Ser314→Asp and Gln645→Glu;
wherein the amino acid numbering refers to the amino acid sequence as shown in SEQ ID NO:2.

Analysis of the amino acid sequence of human blood coagulation Factor V revealed that the amino acids of at least one of the following groups (i) to (iv) of amino acids in the sequence of human blood coagulation Factor V may be substituted:
(i) Leu594, Ser637, Pro642 and Leu647;
(ii) Ser559, Ser641 and Ser1808;
(iii) Lys1773, optionally Glu1862 and Gly235; and/or
(iv) Ser199, Tyr204, Lys286 and Gln590;
wherein the amino acid numbering refers to the amino acid sequence as shown in SEQ ID NO:4.

Preferably, one or more of the following groups (i) to (iv) of substitutions are effected:
(i) Leu594→His, Ser637→Cys, Pro642→His and Leu647→Met;
(ii) Ser559→His, Ser641→Asp and Ser1808→Glu;
(iii) Lys1773→Glu, optionally Glu1862→Asp and Gly235→Glu; and/or
(iv) Ser199→Glu, Tyr204→His, Lys286→Asp and Gln590→Glu;
wherein the amino acid numbering refers to the amino acid sequence as shown in SEQ ID NO:4.

In one embodiment, mutations are introduced in the homologue of human ceruloplasmin, which increase the intrinsic stability of the A2 domain by introducing a novel metal binding site within the A2 domain.

In another embodiment, mutations are introduced in the homologue of human ceruloplasmin, which increase the association of the A2 domain either to the A1 or to the A3 domain of the A1/A3-C1-C2 dimer by the introduction of novel metal binding sites or metal bridges. These modifications convey to the activated form of such Factor VIII mutants a longer functional half-life.

In yet another embodiment, mutations are introduced in the homologue of human ceruloplasmin, which improve the strength of the metal binding site between the A1 domain and the A3 domain of the light chain and also have a longer functional half-life

Another aspect of the invention is a method for increasing the intrinsic stability of a homologue of human ceruloplasmin by increasing the structural stability of the A2 domain by introducing a novel metal binding site within the A2 domain. Yet another aspect of the invention is a method for increasing the functional half life of the homologue of human ceruloplasmin, comprising introducing one or more mutations in the homologue of human ceruloplasmin, which increase the association of the A2 domain either to the A1 or to the A3 domain of the A1/A3-C1-C2 dimer by the introduction of novel metal binding sites or metal bridges. Still another aspect is a method for increasing the functional half life of the homologue of human ceruloplasmin, comprising improving the strength of the metal binding site between the A1 domain and the A3 domain of the light chain. These methods may comprise the same steps as the method for producing a modified homologue of human ceruloplasmin described above.

Another aspect of the invention is a modified homologue of human ceruloplasmin obtainable by the method described hereinbefore.

Yet another aspect of the invention is a modified homologue of human ceruloplasmin, wherein the modified homologue comprises at least one metal binding site (a) that is not present in the wild type-form of the homologue of human ceruloplasmin and (b) that corresponds to a metal binding site present in human ceruloplasmin or to an incomplete metal binding site in human ceruloplasmin.

Preferably, the homologue of human ceruloplasmin is a polypeptide as defined supra. The preferred embodiments of the modified homologue of the invention correspond to the preferred embodiments described hereinbefore with respect to the method of the invention. In particular, the preferred amino acid substitutions correspond to those recited above.

In one embodiment, the invention relates to a modified Factor VIII molecule in which the amino acids Leu649 and Phe697 have been substituted. Leu649 may be replaced with His or Cys, Leu 649 is preferably replaced with His. Phe 697 may be replaced with His or Cys, Phe697 is preferably replaced with His. Most preferably, the following substitutions have been effected: Leu649→His and Phe697→His.

Another embodiment of the invention is a Factor VIII molecule in which the amino acids Ala610 and Asn1950 have been substituted. Ala610 may be replaced with Glu or Asp, Ala610 is preferably replaced with Glu. Asn1950 may be replaced with Glu or Asp, Asn1950 is preferably replaced with Glu. Most preferably, the following substitutions have been effected: Ala610→Glu and Asn1950→Glu.

Another embodiment of the invention is a Factor VIII molecule in which the amino acids Thr263 and optionally Glu2004 have been substituted. Thr263 may be replaced with Glu or Asp, Thr263 is preferably replaced with Glu. Glu2004 may be replaced with Asp. Most preferably, the following substitutions have been effected: Thr263→Glu and Glu2004→Asp.

Another embodiment of the invention is a Factor VIII molecule in which the amino acids Ala227, Ser314 and Gln645 have been substituted. Ala227 may be replaced with Glu or Asp; Ala227 is preferably replaced with Glu. Ser314 may be replaced with Asp or Glu; Ser314 is preferably replaced with Asp. Gln645 may be replaced with Glu or Asp; Gln645 is preferably replaced with Glu. Most preferably, the following substitutions have been effected: Ala227→Glu, Ser314→Asp and Gln645→Glu.

The various embodiments of modified Factor VIII molecules may be combined with each other.

In a further embodiment, the invention relates to a modified Factor V molecule in which the amino acids Leu594, Ser637, Pro642 and Leu647 have been substituted. Leu594 may be replaced with His or Cys; Leu594 is preferably replaced with His. Ser637 may be replaced with Cys or His; Ser637 is preferably replaced with Cys. Pro642 may be replaced with His or Cys; Pro642 is preferably replaced with His. Leu647 is preferably replaced with Met. Most preferably, the following substitutions have been effected: Leu594→His, Ser637→Cys, Pro642→His and Leu647→Met.

Another embodiment of this invention is a modified Factor V molecule in which the amino acids Ser559, Ser641 and Ser1808 have been substituted. Ser559 may be replaced with His or Cys; Ser559 is preferably replaced with His. Ser641 may be replaced with Asp or Glu; Ser641 is preferably replaced with Asp. Ser1808 may be replaced with Glu or Asp; Ser1808 is preferably replaced with Glu. Most preferably, the following substitutions have been effected: Ser559**→**His, Ser641→Asp and Ser1808→Glu.

Another embodiment of this invention is a modified Factor V molecule in which the amino acids Lys1773, Gly235 and optionally Glu1862 have been substituted. Lys1773 may be replaced with Glu or Asp; Lys1773 is preferably replaced with Glu. Gly235 may be replaced with Glu or Asp; Gly235 is preferably replaced with Glu. Glu1862 may be replaced with Asp. Most preferably, the following substitutions have been effected: Lys1773→Glu, Glu1862→Asp and Gly235→Glu.

Another embodiment of this invention is a modified Factor V molecule in which the amino acids Ser199, Tyr204, Lys286 and Gln590 have been substituted. Ser199 may be replaced with Glu or Asp; Ser199 is preferably replaced with Glu. Tyr204 may be replaced with His or Cys; Tyr204 is preferably replaced with His. Lys286 may be replaced with Asp or Glu; Lys286 is preferably replaced with Asp. Gln590 may be replaced with Glu or Asp; Gln590 is preferably replaced with Glu. Most preferably, the following substitutions have been effected: Ser199→Glu, Tyr204→His, Lys286→Asp and Gln590→Glu.

Yet another aspect of the present invention are mutants of FV (which has a structure which is very similar to that of Factor VIII (Kane et al., 1986)) with increased stability. The various embodiments of modified Factor V molecules may be combined with each other.

Generally, the modified homologue of human ceruloplasmin has an increased stability compared to the non-modified form and/or compared to the wild type form of the homologue of human ceruloplasmin. An increase in stability of the mutated Factor VIII molecules can for example be measured as previously described by functional assays (Lollar P et al. J. Biol. Chem. 267:23652-57 (1992)) in which the decay of FVIII activity over time after thrombin activation is described as well as in a physical assay (Persson E et al. Biochemistry 34:12775-81 (1995), Saenko et al. Vox Sang, 2002, 83, pp.89-96) in which the dissociation of the A2 domain can be followed in real time by plasmon surface resonance by attaching FVIII to a surface and subsequent activation by thrombin. Measured is the change in refractive index due to the dissociation of the FVIII trimer.

The stability in terms of reduced dissociation of the A2 domain is usually increased by at least 50%, preferably by at least 100%, more preferably by at least 200%, even more preferably by at least 500% compared to the non-modified form and/or to the wild type form of the homologue of human ceruloplasmin.

In another embodiment, the modified homologue of the invention exhibits an increased functional half-life compared to the non-modified form and/or to the wild type form of the homologue of human ceruloplasmin. The functional half life can be determined in vitro as shown in figure 5 of US 2003/0125232 or as published by Sandberg (Thromb. Haemost. 2001 ;85(1):93-100) and Gale (Gale et al., J. Thromb. Haemost., 2003, 1: p. 1966-1971) which basically consists of determining the kinetics of FVIII activity after thrombin. In vivo one could test the modified FVIII in animal models of hemophilia A, like FVIII knockout mice, in which one would expect a longer lasting hemostatic effect of a stabilized FVIII or a higher hemostatic effect at the same concentration as compared to wild type FVIII. The hemostatic effect could be tested for example by determining time to arrest of bleeding after a tail clip.

The functional half life is usually increased by at least 50%, preferably by at least 100%, more preferably by at least 200%, even more preferably by at least 500% compared to the non-modified form and/or to the wild type form of the homologue of human ceruloplasmin.

The functional half life of the wild type form of human Factor VIIIa is 2.1 minutes. The functional half life of the modified Factor VIIIa molecule of the invention is usually at least about 3.15 minutes, preferably at least about 4.2 minutes, more preferably at least about 6.3 minutes, most preferably at least about 12.6 minutes.

Methods to determine whether a metal binding site has been added to a polypeptide are known to those skilled in the art. For example, it is possible to measure the amount of metal in a homologue of human ceruloplasmin after modification and compare it to that prior to modification. A suitable method is *EPR spectroscopy* (Tagliavacca et al. 1997, JBC, 272: 27428-27434).

The invention further relates to a polynucleotide encoding a modified homologue of human ceruloplasmin as described in this application. The term "polynucleotide(s)" generally refers to any polyribonucleotide or polydeoxyribonucleotide that may be unmodified RNA or DNA or modified RNA or DNA. The polynucleotide may be single- or double-stranded DNA, single or double-stranded RNA. As used herein, the term "polynucleotide(s)" also includes DNAs or RNAs that comprise one or more modified bases and/or unusual bases, such as inosine. It will be appreciated that a variety of modifications may be made to DNA and RNA that serve many useful purposes known to those of skill in the art. The term "polynucleotide(s)" as it is employed herein embraces such chemically, enzymatically or metabolically modified forms of polynucleotides, as well as the chemical forms of DNA and RNA characteristic of viruses and cells, including, for example, simple and complex cells.

The skilled person will understand that, due to the degeneracy of the genetic code, a given polypeptide can be encoded by different polynucleotides. These "variants" are encompassed by this invention.

Preferably, the polynucleotide of the invention is an isolated polynucleotide. The term "isolated" polynucleotide refers to a polynucleotide that is substantially free from other nucleic acid sequences, such as and not limited to other chromosomal and extrachromosomal DNA and RNA. Isolated polynucleotides may be purified from a host cell. Conventional nucleic acid purification methods known to skilled artisans may be used to obtain isolated polynucleotides. The term also includes recombinant polynucleotides and chemically synthesized polynucleotides.

Yet another aspect of the invention is a plasmid or vector comprising a polynucleotide according to the invention. Preferably, the plasmid or vector is an expression vector. In a particular embodiment, the vector is a transfer vector for use in human gene therapy.

Still another aspect of the invention is a host cell comprising a polynucleotide of the invention or a plasmid or vector of the invention.

The host cells of the invention may be employed in a method of producing a modified homologue of human ceruloplasmin, which is part of this invention. The method comprises:
(a) culturing host cells of the invention under conditions such that the modified homologue of human ceruloplasmin is expressed; and
(b) optionally recovering the modified homologue of human ceruloplasmin from the host cells or from the culture medium.

It is preferred to purify the modified homologue of the present invention to ≥ 80% purity, more preferably ≥ 95% purity, and particularly preferred is a pharmaceutically pure state that is greater than 99.9% pure with respect to contaminating macromolecules, particularly other proteins and nucleic acids, and free of infectious and pyrogenic agents. Preferably, an isolated or purified modified homologue of the invention is substantially free of other polypeptides.

The various products of the invention are useful as medicaments. Accordingly, the invention relates to a pharmaceutical composition comprising a modified homologue of human ceruloplasmin as described herein, a polynucleotide of the invention, or a plasmid or vector of the invention.

Another aspect of the invention is the use of a modified homologue of human ceruloplasmin as described herein, of a polynucleotide of the invention, of a plasmid or vector of the invention, or of a host cell of the invention for the manufacture of a medicament for the treatment or prevention of a blood coagulation disorder. Blood coagulation disorders include but are not limited to hemophilia A. Preferably, the treatment comprises human gene therapy.

The invention also concerns a method of treating an individual suffering from a blood coagulation disorder such as hemophilia A. The method comprises administering to said individual an efficient amount of the modified homologue of human ceruloplasmin as described herein. In another embodiment, the method comprises administering to the individual an efficient amount of the polynucleotide of the invention or of a plasmid or vector of the invention. Alternatively, the method may comprise administering to the individual an efficient amount of the host cells of the invention described herein.

### Description of the drawings:

Figure 1: Dendrogram comparing the phylogenetic relation of the A1, A2 and the A3 domains of Factor VIII (FA8), FV (FA5), human ceruloplasmin (CERU) and hephaestin (HEPH) dendrogram. The following program was used for generating the dendrogram: ClustalW Version 1.74. using the 'neighbor joining' algorithm. The parameter "exclude positions with gaps" was on and "Correct for multiple substitutions" was off.
Figure 2: 3D structure of the A1, A2 and the A3 domain of Factor VIII.
Figure 3: Multiple alignments of the inner subdomains of the A1, A2 und A3 domains of Factor VIII. The alignments were done manually. Alternatively ClustalW Version 1.74 with the parameters as in figure 1 can be used.
Figure 4: Multiple alignments of the outer subdomains of the A1, A2 and A3 domains of Factor VIII. The alignment was performed as described with respect to Figure 3.
Figure 5: Figure showing human Ceruloplasmin (PDB Database, entry 1 KCW) with the visualizing program WebLab Viewer 3.7 from "Molecular Simulation Inc). The metal ion in the A2 domain (blue) is coordinated by the amino acids E597, H602, D684 in the A2 domain and E971 in the A3 domain (green). The metal ion in the A3 domain (green) is coordinated by E935, H940, D1025 in the A3 domain and E272 in the A1 domain (red).

### Detailed description of the invention:

### a) General procedure which allows to predict mutations in FVIII and FV and other protein homologous to human cerulosplasmin which enhance their stability by introducing novel metal-binding sites or metal bridges

### Factor VIII structure and related proteins

Factor VIII is closely related to Factor V, which shows a very similar domain structure. The signal peptide is followed by two A-domains. These are followed by the long B-domain, another A-domain and finally by two C-domains. In contrast to the B-domain, both the A and C-domains are also present in other protein families. The B-domain is located between the second and third A-domain, with no homology beyond the Factor VIII family. Each A-domain of Factor VIII consists of two sub-domains, which are related and belong to the Cupredoxin type. But the N-terminal and C-terminal sub-domains of each A-domain belong to very different cupredoxin groups. Either structure information or good (profile-based) sequence analysis were necessary to identify the similarity of both sub-domains. According to database searches, another relative closely related protein is ceruloplasmin. The amino acid sequence of human ceruloplasmin is shown in SEQ ID NO:5. Ceruloplasmin is a member of a small subfamily containing 2 proteins that do have the same close similarity to the coagulation Factors VIII and V. The second protein of this subfamily is Hephaestin. Both proteins of this subfamily have three A-domains, but the insertion of the B-domain and the C-terminal extension of both C-domains are missing. Hephaestin does have a transmembrane-domain at the C-terminus, which cannot be found in ceruloplasmin.

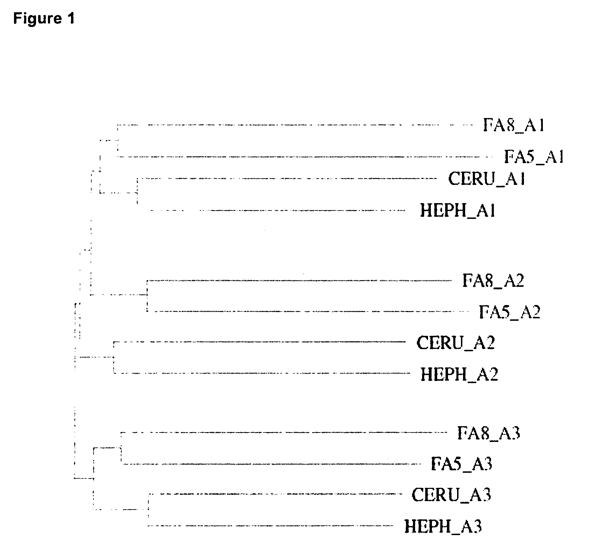

Via a dendrogram-analysis it was found that the A-domains of the Factor VIII / V family and of the ceruloplasmin family group together (figure 1). In particular it was discovered that the A1-domain of Factor VIII is more closely related to the A1-domain of the ceruloplasmin than to the A2- and A3-domains of Factor VIII. The most likely explanation for this is, that the triplication of the A-domain is evolutionary older than the separation of the ceruloplasmin and Factor VIII families. A consequence of the above findings is that the known 3D-structure of ceruloplasmin may be taken for a suitable model for Factor VIII and for activated Factor VIIIa.

### Considerations for stabilization of Factor VIIIa

One problem in the therapeutic application of Factor VIII is the limited half-life of the activated Factor VIII (Factor VIIIa). In Factor VIIIa, the specific A-domains are no longer covalently linked together. As the complex of these three A-domains is not very stable, the half-life of F VIIIa is determined by the rate of dissociation of these domains. Therefore, it was tried to identify mutations based on bioinformatical methods, which could contribute to a moderate stabilization of the activated Factor VIII.

### Structure of Factor VIII and Factor VIIIa

As already discussed above, it was found that the A1-A3-domains of Factor VIIIa are directly related to the A1-A3-domains of ceruloplasmin. Since a highly-resolved crystal structure of ceruloplasmin is available (PDB:1KCW), the structures of the A-domains of Factor VIII and Factor VIIIa can now as a consequence of the invention be derived from such structure.

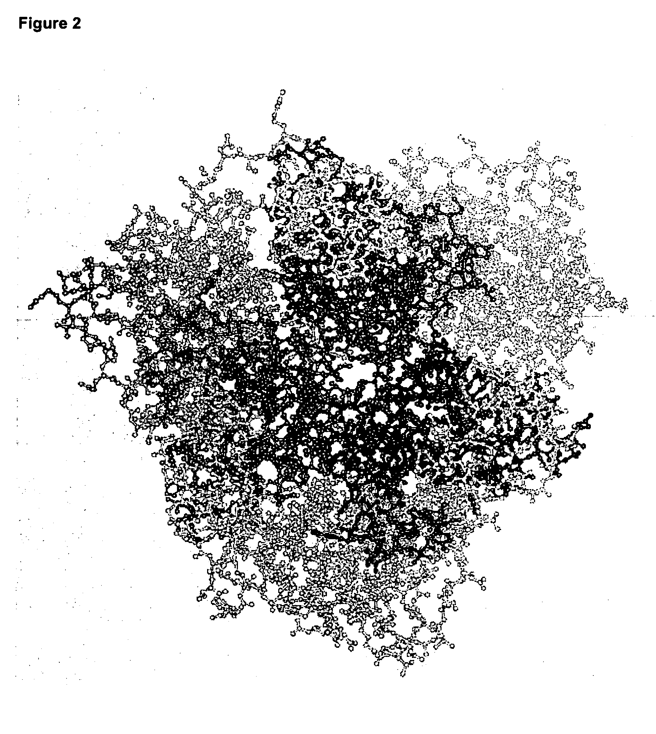

Based on the comparison with the sequence analysis and the structure of ceruloplasmin it was now found for FVIII, that each A-type-domain consists of two distantly related Cupredoxin domains. The three A-domains are then understood to form a pseudo-symmetrical trimer. In each A-domain it then becomes apparent that the C-terminal half-domain is on the inside of the trimer structure and presumably contributes mainly to the trimerization. The N-terminal half domains in this model now made possible build an outside ring in the structure and thereby the N-terminal half domain is also in contact with the C-terminal half domain of the preceding A-domain (figure 2).

In the ceruloplasmin literature, the six half domains are often described as domains d1 to d6, with the even-numbered domains being located inside the trimer and the uneven-numbered being located outside. In order to be better able to superimpose the ceruloplasmin model on the Factor VIII nomenclature, a corresponding nomenclature A1-A3 is used also for ceruloplasmin. For the half domains it is differentiated between A1ₒᵤₜ for the d1-domain which is located outside and A1ᵢₙ for the d2-domain which is located inside. This is similar for A2ₒᵤₜ/A2ᵢₙ and A3ₒᵤₜ/A3ᵢₙ, respectively.

### Contributions of the copper-ions to the stability of ceruloplasmin

Although the inner and outer half domains belong to the cupredoxin-type, they are very different, both in sequence and function. Ceruloplasmin belongs to the "multi-copper oxidases", a class of enzymes, which is defined by a particular arrangement of redox-active copper-ions. Typical multi-copper-oxidases, e. g. ascorbat-oxidase, for which crystallization data are also available, consist of three half domains, two of the "inner" type and one of the "outer" type (because these structures are no trimers, they cannot be termed as "inner" and "outer" trimers). Ceruloplasmin has a more complex structure than other oxidases, and the functional consequences of this difference are not fully understood yet.
Each "inner" sub-domain contains a copper-ion, which is available as Cu²⁺ in the active enzyme and which has the function to oxidize the substrate. This ion is often described as "blue T1-copper". The Cu¹⁺ resulting from this oxidation reaction has to be re-oxidized afterwards. This happens by a special copper/oxygen cluster Cu₃O₂, which is located at the interface of the corresponding "outer" sub-domain and of the preceding inner sub-domain. These copper-ions are often described as "T2/T3" copper. The outer sub-domains do not have any T1-copper positions.

Ceruloplasmin contains three half domains arranged in ring-like form of the "inner" type, each of which contains a T1-copper-ion according to the crystal structure. The T1-ions of the A2ᵢₙ and A3ᵢₙ domains are completely coordinated by the usual four ligands whereas the T1-ion of the A1ᵢₙ domain is only coordinated in triplicate. There is only one T2/T3 cluster at the interfaces of the inner and outer half domains, namely between A1ₒᵤₜ and A3ᵢₙ. The ligand positions at the corresponding positions of the other interfaces are not conserved. These findings on the structure are in accordance with earlier experiments, which lead to the expected 6 copper-ions per ceruloplasmin molecule.

The existing T2/T3 cluster should actually only be able to reoxidize the T1 copper-ion of the A3ᵢₙ domain. Up to now it is not clear, whether the other two T1-positions are catalytically inactive or whether there are alternative possibilities for reoxidization. It has been reported, that at least the T1-copper-ions also have a stabilizing function, besides their redox role. A Cys1021 mutation removed the T1-copper ion out of the A3ᵢₙ sub-domain of ceruloplasmin and resulted in a clearly reduced stability of the molecule (Zaitseva et al., J Biol. Inorg. Chem. 1 pp. 15 (1996)).

In Factor VIII the coordination sites for T1-copper are partly present, while the Cu₃O₂ cluster with the T2/T3 ions is now recognized via the invention as missing (figure 3 and figure 4).
In comparing now which metal-binding sites are present in ceruloplasmin but not in FVIII and FV or other homologous proteins to ceruloplasmin one can identify amino acids which need to be modified to introduce said metal-binding sites also into FVIII, FV or other proteins homologous to ceruloplasmin.

### b) increasing the intrinsic stability of Factor VIII by increasing the structural stability of the A2 domain by introducing a novel metal binding site within the A2 domain.

Based on the previous insights, stabilization of the inner sub-domains is achieved by providing the T1-copper-ions with an optimal coordination. As the loss of the T1-ions causes a dramatic reduction in stability in ceruloplasmin, it would be reasonable to increase the stability of Factor VIII by coordinating the T1-copper-ion by proper ligands for each sub-domain.

### A2

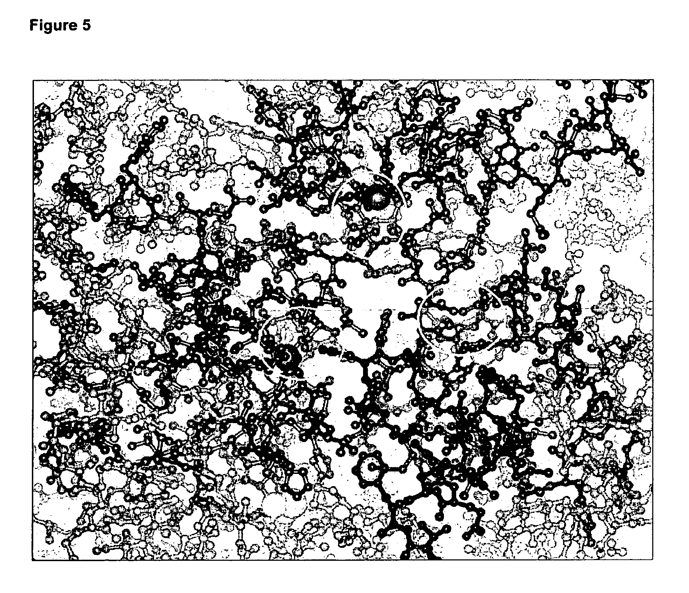

The four amino acids His637, Cys680, His685 and Met690 coordinate the T1-copper-ion inside the sub-domain A2ᵢₙ of the ceruloplasmin. As shown in figure 5, Leu649, Cys692, Phe697 and Met702 occupy the corresponding positions in human Factor VIII. Thus, two amino acids are not able to form a T1-copper coordination site and it can be assumed that the A2ᵢₙ sub-domain in the wild-type-form of Factor VIII does not contain a T1-copper-ion. Thus a Factor VIII mutant which replaces the two amino acids by amino acids which are capable to form such coordination sites is one aspect of the present invention. For example a targeted mutagenesis of Leu649→ His and Phe697→ His should provide the A2 domain with the capability to bind a copper ion and hence stabilize the resulting Factor VIII molecule.

The A1 and A3 domains contain already the necessary amino acids, which allow an optimal coordination of T-1 copper.

### c) Mutationswhich increase the association of the A2 domain either to the A1 or to the A3 domain of the A1/A3-C1-C2 dimer by the introduction of novel metal binding sites or metal bridges

### Contributions of other metal-ions for stabilization of ceruloplasmin

The analysis of the ceruloplasmin-3D-structure leads to another unexpected result. There are two more copper-ions beside the six expected copper-ions in the X-ray structure, which are paradoxically not mentioned in the literature. These ions are each coordinated by four optimal amino acids. In addition, these amino acids are perfectly conserved in the ceruloplasmin-related protein hephaestin. Therefore, the existence of these additional ions in the ceruloplasmin structure cannot be considered as coincidence. One possible explanation is, that these positions are occupied under physiological conditions by other metal-ions (e.g. Ca²⁺), which have been replaced by copper under the crystallization conditions.

Particularly interesting is the position of those additional ions, which are located at the interface of the inner sub-domains (figure 5). One of the ions (residue 42 in the structure 1 KCW. Bielli et al., J Biol Chem. 2001 Jan 26;276(4):2678-85.) is coordinated by three amino acids out of A2ᵢₙ as well as by a glutamic acid out of A3ᵢₙ, the second ion (residue 62) is coordinated by three amino acids out of A3ᵢₙ as well as by a glutamic acid out of A1ᵢₙ. The third analogous position is not occupied by a metal-ion, most likely because one of the coordinated side chains is missing.

The fact that these positions are well conserved in the whole ceruloplasmin family (see figure 3 and 4) suggests, that they may serve a defined function. The position of the ions in the interfaces of the inner sub-domains and the participation of a coordinating glutamic acid-residue of the neighboured domain (figure 5) suggest, that these ions are involved in the stabilization of the A1-A2-A3 trimer.

### Rational design for stabilization of domain interactions

The coordinating amino acids of the bridging metal-ions, which have been identified in ceruloplasmin, are only rudimentary available in Factor VIII. Under the assumption that the trimer interfaces of Factor VIII/VIIIa and ceruloplasmin are based upon similar mechanisms, an artificial generation of analogous metal-bridges could significantly contribute to a stabilization of Factor VIIIa. Optimal coordinating amino acids for this metal-ion appear to be composed of Glu-His-Asp of the first Aᵢₙ domain, as well as of a Glu residue of the proximate Aᵢₙ domain.

### A1 ↔ A2 in FVIII

This interaction could be increased by a metal ion bridge of the A1ᵢₙ domain and a glutamic acid residue of the A2ᵢₙ domain. The ceruloplasmin structure does not contain such an ion, probably because only two of four necessary coordinating amino acids are optimally occupied: Glu236, Tyr241, Asn323 of A1ᵢₙ as well as Glu633 of A2ᵢₙ. The sequence of Factor VIII is Ala227, His232, Ser314 out of A1ᵢₙ as well as Gln645 out of A2ᵢₙ at the corresponding positions. An optimal binding site in FVIII can be obtained by targeted mutagenesis of Ala227→Glu, Ser314→Asp as well as Gln645→Glu.

### A2 ↔ A3

This interaction could be increased by a metal ion bridge between the A2ᵢₙ domain and a glutamic acid residue of the A3ᵢₙ domain. The ceruloplasmin structure contains a corresponding metal-ion (copper, residue 42). The coordinating amino acids are Glu597, His602 and Asp684 of A2ᵢₙ as well as Glu971 of A3ᵢₙ. The sequence of Factor VIII has Ala610, His615, Asp696 of A2ᵢₙ as well as Asn1950 of A3ᵢₙ at the corresponding positions. An optimal binding site in FVIII can be obtained by the targeted mutagenesis of Ala610→Glu as well as Asn1950→Glu.

### d) Mutants, which improve the strength of the metal binding site between the A1 domain and the A3 domain of the light chain and also have a longer functional half-life

### A3 ↔ A1 in FVIII

This interaction could be increased by a metal ion bridge between the A3ᵢₙ domain and a glutamic acid residue of the A1ᵢₙ domain. The ceruloplasmin structure contains a respective metal ion (copper, residue 62). The coordination sites are Glu935, His940 and Asp1025 of A3ᵢₙ as well as Glu272 of A1ᵢₙ. At the corresponding positions, the sequence of Factor VIII has Glu1914, His1919 and Glu2004 of A3ᵢₙ as well as Thr263 of A1ᵢₙ (see figure 4). An optimal binding site in FVIII can be obtained by the targeted mutagenesis of Thr263→Glu as well as Glu2004→Asp.

Another aspect of the present invention are therefore mutants of Factor VIII which are constructed according to the above described rationale and the proposed amino acid exchanges. The proposed amino acids are only examples as homologous amino acids also being able to coordinate metal ions might alternatively be introduced at the indicated positions, e.g. Asp for Glu and/or Cys for His and *vice versa*.

Tagliavacca et al. (JBC (1997) Vol. 272, pp. 27428-27434) tested the relevance of two putative Cu (II) consensus binding sites (His99 and His1957) and two putative Cu (I) consensus sites (Cys310) for secretion efficacy and activity of Factor VIII by site directed mutagenesis and found that Cys310 plays an important role in coordinating Cu (I) whereas His99 and His1957 probably do not bind copper. By applying our bioinformatical analysis we found that His99 and His1957 belong to an incomplete T2/T3 copper-binding site This copper-binding site could then be completed and made functional by mutating Ser1959 to His1959.

### Considerations for the stabilization of FV

### Inner stabilization of the A2 domain of FV

The amino acid sequence of human Factor V is shown in SEQ ID NO:4. By analogy with the analysis of Factor VIII the inner stabilization of the A2 domain of FV can be achieved by introducing the following mutations.
Leu594→His,
Ser637→Cys,
Pro642→His
Leu647**→**Met

### Rational design for stabilization of domain interactions

### A3 <-> A1 for FV

By analogy with the analysis of Factor VIII the interaction between the A3 and the A1 domain of FV can be stabilized by introducing the following mutations.
Lys1773→Glu,
optionally Glul862→Asp,
Gly235→Glu

### A1 <-> A2 for FV

By analogy with the analysis of Factor VIII the interaction between the A2 and the A1 domain of FV can be stabilized by introducing the following mutations.
Ser199→Glu.
Tyr204→His
Lys286→Asp
Gln590→Glu

### A2 <-> A3 for FV

By analogy with the analysis of Factor VIII the interaction between the A3 and the A2 domain of FV can be stabilized by introducing the following mutations.
Ser559→His
Ser641**→**Asp
Ser1808→Glu

The amino acids forming three metal binding sites and one incomplete metal binding site in human ceruloplasmin are shown in the following tables. The corresponding amino acids in the Factor V ("WT FV") and in the Factor VIII ("WT FVIII") amino acid sequence are also shown. The tables further summarize possible and preferred mutations in the Factor V ("Mut. FV") and Factor VIII ("Mut. FVIII") sequences. Preferred mutations are shaded and printed in bold.

**Table 1.**

| **Intra-subunit binding sites:** | | | | |
|---|---|---|---|---|
| A2 | | | | |
| Ceruloplasmin | His637 | Cys680 | His685 | Met690 |
| WT FVIII | Leu649 | Cys692 | Phe697 | Met702 |
| Mut. FVIII | **His649** | | **His697** | |
| | Cys649 | | Cys697 | |
| WT FV | Leu594 | Ser637 | Pro642 | Leu647 |
| Mut. FV | **His594** | **Cys637** | **His642** | **Met647** |
| | Cys594 | His637 | Cys642 | |

**Table 2.**

| **Inter-Subunit binding sites:** | | | | |
|---|---|---|---|---|
| A3-A2 | | | | |
| Ceruloplasmin | Glu597 | His602 | Asp684 | Glu971 |
| WT FVIII | Ala610 | His615 | Asp696 | Asn1950 |
| Mut. FVIII | **Glu610** | | | **Glu1950** |
| | Asp610 | | | Asp1950 |
| WT FV | Glu554 | Ser559 | Ser641 | Ser1808 |
| Mut. FV | | **His559** | **Asp641** | **Glu1808** |
| | | Cys559 | Glu641 | Asp1808 |
| | | | | |

| A3-A1 | | | | |
|---|---|---|---|---|
| Ceruloplasmin | Glu935 | His940 | Asp1025 | Glu272 |
| WT FVIII | Glu1914 | His1919 | Glu2004 | Thr263 |
| Mut. FVIII | | | **Asp2004** | **Glu263** |
| | | | | Asp263 |
| WT FV | Lys1773 | His1778 | Glu1862 | Gly235 |
| Mut. FV | **Glu1773** | | **Asp1862** | **Glu235** |
| | Asp1773 | | | Asp235 |
| | | | | |

| A 1-A2 | | | | |
|---|---|---|---|---|
| Ceruloplasmin | Glu236 | Tyr241 | Asn323 | Glu633 |
| WT FVIII | Ala227 | His232 | Ser314 | Gln645 |
| Mut. FVIII | **Glu227** | | **Asp314** | **Glu645** |
| | Asp227 | | Glu314 | Asp645 |
| WT FV | Ser199 | Tyr204 | Lys286 | Gln590 |
| Mut. FV | **Glu199** | **His204** | **Asp286** | **Glu590** |
| | Asp199 | Cys204 | Glu286 | Asp590 |

### Expression of the proposed mutants

The production of recombinant mutant proteins at high levels in suitable host cells requires the assembly of the above-mentioned modified cDNAs into efficient transcriptional units together with suitable regulatory elements in a recombinant expression vector that can be propagated in various expression systems according to methods known to those skilled in the art. Efficient transcriptional regulatory elements could be derived from viruses having animal cells as their natural hosts or from the chromosomal DNA of animal cells. Preferably, promoter-enhancer combinations derived from the Simian Virus 40, adenovirus, BK polyoma virus, human cytomegalovirus, or the long terminal repeat of Rous sarcoma virus, or promoter-enhancer combinations including strongly constitutively transcribed genes in animal cells like beta-actin or GRP78 can be used. In order to achieve stable high levels of mRNA transcribed from the cDNAs, the transcriptional unit should contain in its 3'-proximal part a DNA region encoding a transcriptional termination-polyadenylation sequence. Preferably, this sequence is derived from the Simian Virus 40 early transcriptional region, the rabbit beta-globin gene, or the human tissue plasminogen activator gene.

The cDNAs are then integrated into the genome of a suitable host cell line for expression of the Factor VIII proteins. Preferably this cell line should be an animal cell-line of vertebrate origin in order to ensure correct folding, disulfide bond formation, asparagine-linked glycosylation and other post-translational modifications as well as secretion into the cultivation medium. Examples on other post-translational modifications are tyrosine O-sulfation, and proteolytic processing of the nascent polypeptide chain. Examples of cell lines that can be use are monkey COS-cells, mouse L-cells, mouse C127-cells, hamster BHK-21 cells, human embryonic kidney 293 cells, and preferentially hamster CHO-cells.

The recombinant expression vector encoding the corresponding cDNAs can be introduced into an animal cell line in several different ways. For instance, recombinant expression vectors can be created from vectors based on different animal viruses. Examples of these are vectors based on baculovirus, vaccinia virus, adenovirus, and preferably bovine papilloma virus.

The transcription units encoding the corresponding DNA's can also be introduced into animal cells together with another recombinant gene which may function as a dominant selectable marker in these cells in order to facilitate the isolation of specific cell clones which have integrated the recombinant DNA into their genome. Examples of this type of dominant selectable marker genes are Tn5 amino glycoside phosphotransferase, conferring resistance to geneticin (G418), hygromycin phosphotransferase, conferring resistance to hygromycin, and puromycin acetyl transferase, conferring resistance to puromycin. The recombinant expression vector encoding such a selectable marker can reside either on the same vector as the one encoding the cDNA of the desired protein, or it can be encoded on a separate vector which is simultaneously introduced and integrated to the genome of the host cell, frequently resulting in a tight physical linkage between the different transcription units.

Other types of selectable marker genes which can be used together with the cDNA of the desired protein are based on various transcription units encoding dihydrofolate reductase (dhfr). After introduction of this type of gene into cells lacking endogenous dhfr-activity, preferentially CHO-cells (DUKX-B11, DG-44) it will enable these to grow in media lacking nucleosides. An example of such a medium is Ham's F12 without hypoxanthine, thymidin, and glycine. These dhfr-genes can be introduced together with the Factor VIII cDNA transcriptional units into CHO-cells of the above type, either linked on the same vector or on different vectors, thus creating dhfr-positive cell lines producing recombinant protein.

If the above cell lines are grown in the presence of the cytotoxic dhfr-inhibitor methotrexate, new cell lines resistant to methotrexate will emerge. These cell lines may produce recombinant protein at an increased rate due to the amplified number of linked dhfr and the desired protein's transcriptional units. When propagating these cell lines in increasing concentrations of methotrexate (1-10000 nM), new cell lines can be obtained which produce the desired protein at very high rate.

The above cell lines producing the desired protein can be grown on a large scale, either in suspension culture or on various solid supports. Examples of these supports are micro carriers based on dextran or collagen matrices, or solid supports in the form of hollow fibres or various ceramic materials. When grown in cell suspension culture or on micro carriers the culture of the above cell lines can be performed either as a bath culture or as a perfusion culture with continuous production of conditioned medium over extended periods of time. Thus, according to the present invention, the above cell lines are well suited for the development of an industrial process for the production of the desired recombinant mutant proteins

The recombinant mutant protein, which accumulates in the medium of secreting cells of the above types, can be concentrated and purified by a variety of biochemical and chromatographic methods, including methods utilizing differences in size, charge, hydrophobicity, solubility, specific affinity, etc. between the desired protein and other substances in the cell cultivation medium.

An example of such purification is the adsorption of the recombinant mutant protein to a monoclonal antibody which is immobilised on a solid support. After desorption, the protein can be further purified by a variety of chromatographic techniques based on the above properties.

The recombinant proteins described in this invention can be formulated into pharmaceutical preparations for therapeutic use. The purified proteins may be dissolved in conventional physiologically compatible aqueous buffer solutions to which there may be added, optionally, pharmaceutical excipients to provide pharmaceutical preparations.

The modified polynucleotides (e.g. DNA) of this invention may also be integrated into a transfer vector for use in the human gene therapy.

The various embodiments described herein may be combined with each other. The present invention will be further described more in detail in the following examples thereof. This description of specific embodiments of the invention will be made in conjunction with the appended figures.

### Examples:

### Generation of Factor VIII mutants

For the generation of Factor VIII mutants, Factor VIII cDNA or suitable sub fragments are first sub cloned into a suitable cloning vector to reduce subsequent sequencing efforts. Site directed mutagenesis is then performed with a commercially available mutagenesis kit (e.g. Stratagene QuickChange SiteDirected Mutagenesis Kit) according to the manufacturer's instructions. Primers used for mutagenesis are listed below, where the mutagenic bases are indicated in bold letters.

### Mutation: L649H

forward primer 5'GGAGCACAGACTGACTTCCACTCTGTCTTCTTCTCTGG3'
(SEQ ID NO:6)

reverse primer 5´CCAGAGAAGAAGACAGAGTGGAAGTCAGTCTGTGCTCC3´
(SEQ ID NO:7)

### Mutation: F697H

forward primer 5'GGTGCCACAACTCAGACCATCGGAACAGAGGCATG3'
(SEQ ID NO:8)

reverse primer 5'CATGCCTCTGTTCCGATGGTCTGAGTTGTGGCACC3'
(SEQ ID NO:9)

### Mutation: T263E

forward primer
5'GTGATTGGAATGGGCACCGAGCCTGAAGTGCACTCAATATTCCTC3'
(SEQ ID NO:10)

reverse primer
5'GAGGAATATTGAGTGCACTTCAGGCTCGGTGCCCATTCCAATCAC3'
(SEQ ID NO:11)

### Mutation: E2004D

forward primer 5'GAATGCCTTATTGGCGACCATCTACATGCTGGG3'
(SEQ ID NO:12)

reverse primer 5'CCCAGCATGTAGATGGTCGCCAATAAGGCATTC3'
(SEQ ID NO:13)

### Mutation: A227E

forward primer 5'GATGCTGCATCTGCTCGGGAGTGGCCTAAAATGCAC3'
(SEQ ID NO:14)

reverse primer 5'GTGCATTTTAGGCCACTCCCGAGCAGATGCAGCATC3'
(SEQ ID NO:15)

### Mutation: S314D

forward primer
5′CTACTGTTTTGTCATATCTCTGACCACCAACATGATGGCATGG3′
(SEQ ID NO:16)

reverse primer
5′CCATGCCATCATGTTGGTGGTCAGAGATATGACAAAACAGTAG3′
(SEQ ID NO:17)

### Mutation: Q645E

forward primer 5'CATTCTAAGCATTGGAGCAGAGACTGACTTCCTTTCTG3'
(SEQ ID NO:18)

reverse primer 5'CAGAAAGGAAGTCAGTCTCTGCTCCAATGCTTAGAATG3'
(SEQ ID NO:19)

### Mutation: A610E

forward primer
5′GAGGATCCAGAGTTCCAAGAGTCCAACATCATGCACAGC3′
(SEQ ID NO:20)

reverse primer
5'GCTGTGCATGATGTTGGACTCTTGGAACTCTGGATCCTC3'
(SEQ ID NO:21)

### Mutation: N1950E

forward primer
5'GCTCAGCATGGGCAGCGAGGAAAACATCCATTCTATTCATTTC3'
(SEQ ID NO:22)

reverse primer
5'GAAATGAATAGAATGGATGTTTTCCTCGCTGCCCATGCTGAGC3'
(SEQ ID NO:23)

After clone isolation and sequence verification mutant subfragments are reinserted into the respective expression vector.

### Expression of Factor VIII mutants

Transfection of Factor VIII mutant clones and expression of the mutant Factor VIII molecules is done as described previously and known to those skilled in the art (e.g. Plantier JL et al. Thromb. Haemost. 86:596-603 (2001)). Purification of recombinant Factor VIII is reviewed in Boedeker BDG (Seminars Thromb. Haemost. 27:385-394 (2001)).

Pro Pro Leu Leu Thr Arg Tyr Leu Arg Ile His Pro Gln Ser Trp
    2300                    2305                    2310

gtg cac cag att gcc ctg agg atg gag gtt ctg ggc tgc gag gca        6984
Val His Gln Ile Ala Leu Arg Met Glu Val Leu Gly Cys Glu Ala
    2315                    2320                    2325

cag gac ctc tac                                                    6996
Gln Asp Leu Tyr
    2330


<210>  2
<211>  2332
<212>  PRT
<213>  homo sapiens

<400>  2

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

Gln Asp Leu Tyr
    2330

<210> 3
<211> 6588
<212> DNA
<213> Homo sapiens

<220>
<221> CDS
<222> (1)..(6588)

<400> 3
gca cag cta agg cag ttc tac gtg gct gct cag ggc atc agt tgg agc      48
Ala Gln Leu Arg Gln Phe Tyr Val Ala Ala Gln Gly Ile Ser Trp Ser
1               5                   10                  15

tac cga cct gag ccc aca aac tca agt ttg aat ctt tct gta act tcc      96
Tyr Arg Pro Glu Pro Thr Asn Ser Ser Leu Asn Leu Ser Val Thr Ser
            20                  25                  30

ttt aag aaa att gtc tac aga gag tat gaa cca tat ttt aag aaa gaa      144
Phe Lys Lys Ile Val Tyr Arg Glu Tyr Glu Pro Tyr Phe Lys Lys Glu
        35                  40                  45

aaa cca caa tct acc att tca gga ctt ctt ggg cct act tta tat gct      192
Lys Pro Gln Ser Thr Ile Ser Gly Leu Leu Gly Pro Thr Leu Tyr Ala
    50                  55                  60

gaa gtc gga gac atc ata aaa gtt cac ttt aaa aat aag gca gat aag      240
Glu Val Gly Asp Ile Ile Lys Val His Phe Lys Asn Lys Ala Asp Lys
65                  70                  75                  80

ccc ttg agc atc cat cct caa gga att agg tac agt aaa tta tca gaa      288
Pro Leu Ser Ile His Pro Gln Gly Ile Arg Tyr Ser Lys Leu Ser Glu
                85                  90                  95

ggt gct tct tac ctt gac cac aca ttc cct gca gag aag atg gac gac      336
Gly Ala Ser Tyr Leu Asp His Thr Phe Pro Ala Glu Lys Met Asp Asp
            100                 105                 110

gct gtg gct cca ggc cga gaa tac acc tat gaa tgg agt atc agt gag      384
Ala Val Ala Pro Gly Arg Glu Tyr Thr Tyr Glu Trp Ser Ile Ser Glu
        115                 120                 125

gac agt gga ccc acc cat gat gac cct cca tgc ctc aca cac atc tat      432
Asp Ser Gly Pro Thr His Asp Asp Pro Pro Cys Leu Thr His Ile Tyr
    130                 135                 140

tac tcc cat gaa aat ctg atc gag gat ttc aac tct ggg ctg att ggg      480
Tyr Ser His Glu Asn Leu Ile Glu Asp Phe Asn Ser Gly Leu Ile Gly
145                 150                 155                 160

ccc ctg ctt atc tgt aaa aaa ggg acc cta act gag ggt ggg aca cag      528
Pro Leu Leu Ile Cys Lys Lys Gly Thr Leu Thr Glu Gly Gly Thr Gln
                165                 170                 175

aag acg ttt gac aag caa atc gtg cta cta ttt gct gtg ttt gat gaa      576
Lys Thr Phe Asp Lys Gln Ile Val Leu Leu Phe Ala Val Phe Asp Glu
            180                 185                 190

agc aag agc tgg agc cag tca tca tcc cta atg tac aca gtc aat gga      624
Ser Lys Ser Trp Ser Gln Ser Ser Ser Leu Met Tyr Thr Val Asn Gly
        195                 200                 205

cgt ctg aat gcc cag gga cgt gtg aat gcc tgg caa gcc aag gca 6264
Arg Leu Asn Ala Gln Gly Arg Val Asn Ala Trp Gln Ala Lys Ala
2075 2080 2085

aac aac aat aag cag tgg cta gaa att gat cta ctc aag atc aag 6309
Asn Asn Asn Lys Gln Trp Leu Glu Ile Asp Leu Leu Lys Ile Lys
2090 2095 2100

aag ata acg gca att ata aca cag ggc tgc aag tct ctg tcc tct 6354
Lys Ile Thr Ala Ile Ile Thr Gln Gly Cys Lys Ser Leu Ser Ser
2105 2110 2115

gaa atg tat gta aag agc tat acc atc cac tac agt gag cag gga 6399
Glu Met Tyr Val Lys Ser Tyr Thr Ile His Tyr Ser Glu Gln Gly
2120 2125 2130

gtg gaa tgg aaa cca tac agg ctg aaa tcc tcc atg gtg gac aag 6444
Val Glu Trp Lys Pro Tyr Arg Leu Lys Ser Ser Met Val Asp Lys
2135 2140 2145

att ttt gaa gga aat act aat acc aaa gga cat gtg aag aac ttt 6489
Ile Phe Glu Gly Asn Thr Asn Thr Lys Gly His Val Lys Asn Phe
2150 2155 2160

ttc aac ccc cca atc att tcc agg ttt atc cgt gtc att cct aaa 6534
Phe Asn Pro Pro Ile Ile Ser Arg Phe Ile Arg Val Ile Pro Lys
2165 2170 2175

aca tgg aat caa agt att gca ctt cgc ctg gaa ctc ttt ggc tgt 6579
Thr Trp Asn Gln Ser Ile Ala Leu Arg Leu Glu Leu Phe Gly Cys
2180 2185 2190

gat att tac 6588
Asp Ile Tyr
2195

<210> 4
<211> 2196
<212> PRT
<213> Homo sapiens

<400> 4

Ala Gln Leu Arg Gln Phe Tyr Val Ala Ala Gln Gly Ile Ser Trp Ser
1               5                   10                  15

Tyr Arg Pro Glu Pro Thr Asn Ser Ser Leu Asn Leu Ser Val Thr Ser
            20                  25                  30

Phe Lys Lys Ile Val Tyr Arg Glu Tyr Glu Pro Tyr Phe Lys Lys Glu
        35                  40                  45

Lys Pro Gln Ser Thr Ile Ser Gly Leu Leu Gly Pro Thr Leu Tyr Ala
    50                  55                  60

Glu Val Gly Asp Ile Ile Lys Val His Phe Lys Asn Lys Ala Asp Lys
65                  70                  75                  80

Pro Leu Ser Ile His Pro Gln Gly Ile Arg Tyr Ser Lys Leu Ser Glu
                85                  90                  95

ggagcacaga ctgacttcca ctctgtcttc ttctctgg                                38

<210>  7
<211>  38
<212>  DNA
<213>  artificial sequence

<220>
<223>  L649H reverse primer

<400>  7
ccagagaaga agacagagtg gaagtcagtc tgtgctcc                                38

<210>  8
<211>  35
<212>  DNA
<213>  artificial sequence

<220>
<223>  F697H forward primer

<400>  8
ggtgccacaa ctcagaccat cggaacagag gcatg                                   35

<210>  9
<211>  35
<212>  DNA
<213>  artificial sequence

<220>
<223>  F697H reverse primer

<400>  9
catgcctctg ttccgatggt ctgagttgtg gcacc                                   35

<210>  10
<211>  45
<212>  DNA
<213>  artificial sequence

<220>
<223>  T263E forward primer

<400>  10
gtgattggaa tgggcaccga gcctgaagtg cactcaatat tcctc                        45

<210>  11
<211>  45
<212>  DNA
<213>  artificial sequence

<220>
<223>  T263E reverse primer

<400>  11
gaggaatatt gagtgcactt caggctcggt gcccattcca atcac                        45

<210>  12
<211>  33
<212>  DNA
<213>  artificial sequence

<220>

<223>  E2004D forward primer

<400>  12
gaatgcctta ttggcgacca tctacatgct ggg                                    33


<210>  13
<211>  33
<212>  DNA
<213>  artificial sequence

<220>
<223>  E2004D reverse primer

<400>  13
cccagcatgt agatggtcgc caataaggca ttc                                    33


<210>  14
<211>  36
<212>  DNA
<213>  artificial sequence

<220>
<223>  A227E forward primer

<400>  14
gatgctgcat ctgctcggga gtggcctaaa atgcac                                 36


<210>  15
<211>  36
<212>  DNA
<213>  artificial sequence

<220>
<223>  A227E reverse primer

<400>  15
gtgcatttta ggccactccc gagcagatgc agcatc                                 36


<210>  16
<211>  43
<212>  DNA
<213>  artificial sequence

<220>
<223>  S314D forward sequence

<400>  16
ctactgtttt gtcatatctc tgaccaccaa catgatggca tgg                         43


<210>  17
<211>  43
<212>  DNA
<213>  artificial sequence

<220>
<223>  S314D reverse primer

<400>  17
ccatgccatc atgttggtgg tcagagatat gacaaaacag tag                         43


<210>  18
<211>  38
<212>  DNA

<213>  artificial sequence

<220>
<223>  Q645E forward primer

<400>  18
cattctaagc attggagcag agactgactt cctttctg                                38


<210>  19
<211>  38
<212>  DNA
<213>  artificial sequence

<220>
<223>  Q645E reverse primer

<400>  19
cagaaaggaa gtcagtctct gctccaatgc ttagaatg                                38


<210>  20
<211>  39
<212>  DNA
<213>  artificial sequence

<220>
<223>  A610E forward primer

<400>  20
gaggatccag agttccaaga gtccaacatc atgcacagc                               39


<210>  21
<211>  39
<212>  DNA
<213>  artificial sequence

<220>
<223>  A610E reverse primer

<400>  21
gctgtgcatg atgttggact cttggaactc tggatcctc                               39


<210>  22
<211>  43
<212>  DNA
<213>  artificial sequence

<220>
<223>  N1950E forward primer

<400>  22
gctcagcatg ggcagcgagg aaaacatcca ttctattcat ttc                          43


<210>  23
<211>  43
<212>  DNA
<213>  artificial sequence

<220>
<223>  N1950E reverse primer

<400>  23
gaaatgaata gaatggatgt tttcctcgct gcccatgctg agc                          43

SEQUENCE LISTING

<110>  Aventis Behring GmbH

<120>  Modified coagulation Factors with enhanced stability and their
       derivatives

<130>  A87

<160>  23

<170>  PatentIn version 3.2

<210>  1
<211>  6996
<212>  DNA
<213>  homo sapiens


<220>
<221>  CDS
<222>  (1)..(6996)

<400>  1
gcc acc aga aga tac tac ctg ggt gca gtg gaa ctg tca tgg gac tat        48
Ala Thr Arg Arg Tyr Tyr Leu Gly Ala Val Glu Leu Ser Trp Asp Tyr
1               5                   10                  15

atg caa agt gat ctc ggt gag ctg cct gtg gac gca aga ttt cct cct        96
Met Gln Ser Asp Leu Gly Glu Leu Pro Val Asp Ala Arg Phe Pro Pro
            20                  25                  30

aga gtg cca aaa tct ttt cca ttc aac acc tca gtc gtg tac aaa aag       144
Arg Val Pro Lys Ser Phe Pro Phe Asn Thr Ser Val Val Tyr Lys Lys
        35                  40                  45

act ctg ttt gta gaa ttc acg gat cac ctt ttc aac atc gct aag cca       192
Thr Leu Phe Val Glu Phe Thr Asp His Leu Phe Asn Ile Ala Lys Pro
    50                  55                  60

agg cca ccc tgg atg ggt ctg cta ggt cct acc atc cag gct gag gtt       240
Arg Pro Pro Trp Met Gly Leu Leu Gly Pro Thr Ile Gln Ala Glu Val
65                  70                  75                  80

tat gat aca gtg gtc att aca ctt aag aac atg gct tcc cat cct gtc       288
Tyr Asp Thr Val Val Ile Thr Leu Lys Asn Met Ala Ser His Pro Val
                85                  90                  95

agt ctt cat gct gtt ggt gta tcc tac tgg aaa gct tct gag gga gct       336
Ser Leu His Ala Val Gly Val Ser Tyr Trp Lys Ala Ser Glu Gly Ala
            100                 105                 110

gaa tat gat gat cag acc agt caa agg gag aaa gaa gat gat aaa gtc       384
Glu Tyr Asp Asp Gln Thr Ser Gln Arg Glu Lys Glu Asp Asp Lys Val
        115                 120                 125

ttc cct ggt gga agc cat aca tat gtc tgg cag gtc ctg aaa gag aat       432
Phe Pro Gly Gly Ser His Thr Tyr Val Trp Gln Val Leu Lys Glu Asn
    130                 135                 140

ggt cca atg gcc tct gac cca ctg tgc ctt acc tac tca tat ctt tct       480
Gly Pro Met Ala Ser Asp Pro Leu Cys Leu Thr Tyr Ser Tyr Leu Ser
145                 150                 155                 160

cat gtg gac ctg gta aaa gac ttg aat tca ggc ctc att gga gcc cta       528
His Val Asp Leu Val Lys Asp Leu Asn Ser Gly Leu Ile Gly Ala Leu
                165                 170                 175

cta gta tgt aga gaa ggg agt ctg gcc aag gaa aag aca cag acc ttg       576

Leu Val Cys Arg Glu Gly Ser Leu Ala Lys Glu Lys Thr Gln Thr Leu
            180                 185                 190

cac aaa ttt ata cta ctt ttt gct gta ttt gat gaa ggg aaa agt tgg      624
His Lys Phe Ile Leu Leu Phe Ala Val Phe Asp Glu Gly Lys Ser Trp
        195                 200                 205

cac tca gaa aca aag aac tcc ttg atg cag gat agg gat gct gca tct      672
His Ser Glu Thr Lys Asn Ser Leu Met Gln Asp Arg Asp Ala Ala Ser
    210                 215                 220

gct cgg gcc tgg cct aaa atg cac aca gtc aat ggt tat gta aac agg      720
Ala Arg Ala Trp Pro Lys Met His Thr Val Asn Gly Tyr Val Asn Arg
225                 230                 235                 240

tct ctg cca ggt ctg att gga tgc cac agg aaa tca gtc tat tgg cat      768
Ser Leu Pro Gly Leu Ile Gly Cys His Arg Lys Ser Val Tyr Trp His
                245                 250                 255

gtg att gga atg ggc acc act cct gaa gtg cac tca ata ttc ctc gaa      816
Val Ile Gly Met Gly Thr Thr Pro Glu Val His Ser Ile Phe Leu Glu
            260                 265                 270

ggt cac aca ttt ctt gtg agg aac cat cgc cag gcg tcc ttg gaa atc      864
Gly His Thr Phe Leu Val Arg Asn His Arg Gln Ala Ser Leu Glu Ile
        275                 280                 285

tcg cca ata act ttc ctt act gct caa aca ctc ttg atg gac ctt gga      912
Ser Pro Ile Thr Phe Leu Thr Ala Gln Thr Leu Leu Met Asp Leu Gly
    290                 295                 300

cag ttt cta ctg ttt tgt cat atc tct tcc cac caa cat gat ggc atg      960
Gln Phe Leu Leu Phe Cys His Ile Ser Ser His Gln His Asp Gly Met
305                 310                 315                 320

gaa gct tat gtc aaa gta gac agc tgt cca gag gaa ccc caa cta cga     1008
Glu Ala Tyr Val Lys Val Asp Ser Cys Pro Glu Glu Pro Gln Leu Arg
                325                 330                 335

atg aaa aat aat gaa gaa gcg gaa gac tat gat gat gat ctt act gat     1056
Met Lys Asn Asn Glu Glu Ala Glu Asp Tyr Asp Asp Asp Leu Thr Asp
            340                 345                 350

tct gaa atg gat gtg gtc agg ttt gat gat gac aac tct cct tcc ttt     1104
Ser Glu Met Asp Val Val Arg Phe Asp Asp Asp Asn Ser Pro Ser Phe
        355                 360                 365

atc caa att cgc tca gtt gcc aag aag cat cct aaa act tgg gta cat     1152
Ile Gln Ile Arg Ser Val Ala Lys Lys His Pro Lys Thr Trp Val His
    370                 375                 380

tac att gct gct gaa gag gag gac tgg gac tat gct ccc tta gtc ctc     1200
Tyr Ile Ala Ala Glu Glu Glu Asp Trp Asp Tyr Ala Pro Leu Val Leu
385                 390                 395                 400

gcc ccc gat gac aga agt tat aaa agt caa tat ttg aac aat ggc cct     1248
Ala Pro Asp Asp Arg Ser Tyr Lys Ser Gln Tyr Leu Asn Asn Gly Pro
                405                 410                 415

cag cgg att ggt agg aag tac aaa aaa gtc cga ttt atg gca tac aca     1296
Gln Arg Ile Gly Arg Lys Tyr Lys Lys Val Arg Phe Met Ala Tyr Thr
            420                 425                 430

gat gaa acc ttt aag act cgt gaa gct att cag cat gaa tca gga atc     1344
Asp Glu Thr Phe Lys Thr Arg Glu Ala Ile Gln His Glu Ser Gly Ile
        435                 440                 445

ttg gga cct tta ctt tat ggg gaa gtt gga gac aca ctg ttg att ata     1392
Leu Gly Pro Leu Leu Tyr Gly Glu Val Gly Asp Thr Leu Leu Ile Ile

450                 455                 460

ttt aag aat caa gca agc aga cca tat aac atc tac cct cac gga atc      1440
Phe Lys Asn Gln Ala Ser Arg Pro Tyr Asn Ile Tyr Pro His Gly Ile
465                 470                 475                 480

act gat gtc cgt cct ttg tat tca agg aga tta cca aaa ggt gta aaa      1488
Thr Asp Val Arg Pro Leu Tyr Ser Arg Arg Leu Pro Lys Gly Val Lys
                485                 490                 495

cat ttg aag gat ttt cca att ctg cca gga gaa ata ttc aaa tat aaa      1536
His Leu Lys Asp Phe Pro Ile Leu Pro Gly Glu Ile Phe Lys Tyr Lys
            500                 505                 510

tgg aca gtg act gta gaa gat ggg cca act aaa tca gat cct cgg tgc      1584
Trp Thr Val Thr Val Glu Asp Gly Pro Thr Lys Ser Asp Pro Arg Cys
        515                 520                 525

ctg acc cgc tat tac tct agt ttc gtt aat atg gag aga gat cta gct      1632
Leu Thr Arg Tyr Tyr Ser Ser Phe Val Asn Met Glu Arg Asp Leu Ala
    530                 535                 540

tca gga ctc att ggc cct ctc ctc atc tgc tac aaa gaa tct gta gat      1680
Ser Gly Leu Ile Gly Pro Leu Leu Ile Cys Tyr Lys Glu Ser Val Asp
545                 550                 555                 560

caa aga gga aac cag ata atg tca gac aag agg aat gtc atc ctg ttt      1728
Gln Arg Gly Asn Gln Ile Met Ser Asp Lys Arg Asn Val Ile Leu Phe
                565                 570                 575

tct gta ttt gat gag aac cga agc tgg tac ctc aca gag aat ata caa      1776
Ser Val Phe Asp Glu Asn Arg Ser Trp Tyr Leu Thr Glu Asn Ile Gln
            580                 585                 590

cgc ttt ctc ccc aat cca gct gga gtg cag ctt gag gat cca gag ttc      1824
Arg Phe Leu Pro Asn Pro Ala Gly Val Gln Leu Glu Asp Pro Glu Phe
        595                 600                 605

caa gcc tcc aac atc atg cac agc atc aat ggc tat gtt ttt gat agt      1872
Gln Ala Ser Asn Ile Met His Ser Ile Asn Gly Tyr Val Phe Asp Ser
    610                 615                 620

ttg cag ttg tca gtt tgt ttg cat gag gtg gca tac tgg tac att cta      1920
Leu Gln Leu Ser Val Cys Leu His Glu Val Ala Tyr Trp Tyr Ile Leu
625                 630                 635                 640

agc att gga gca cag act gac ttc ctt tct gtc ttc ttc tct gga tat      1968
Ser Ile Gly Ala Gln Thr Asp Phe Leu Ser Val Phe Phe Ser Gly Tyr
                645                 650                 655

acc ttc aaa cac aaa atg gtc tat gaa gac aca ctc acc cta ttc cca      2016
Thr Phe Lys His Lys Met Val Tyr Glu Asp Thr Leu Thr Leu Phe Pro
            660                 665                 670

ttc tca gga gaa act gtc ttc atg tcg atg gaa aac cca ggt cta tgg      2064
Phe Ser Gly Glu Thr Val Phe Met Ser Met Glu Asn Pro Gly Leu Trp
        675                 680                 685

att ctg ggg tgc cac aac tca gac ttt cgg aac aga ggc atg acc gcc      2112
Ile Leu Gly Cys His Asn Ser Asp Phe Arg Asn Arg Gly Met Thr Ala
    690                 695                 700

tta ctg aag gtt tct agt tgt gac aag aac act ggt gat tat tac gag      2160
Leu Leu Lys Val Ser Ser Cys Asp Lys Asn Thr Gly Asp Tyr Tyr Glu
705                 710                 715                 720

gac agt tat gaa gat att tca gca tac ttg ctg agt aaa aac aat gcc      2208
Asp Ser Tyr Glu Asp Ile Ser Ala Tyr Leu Leu Ser Lys Asn Asn Ala
                725                 730                 735

att gaa cca aga agc ttc tcc cag aat tca aga cac cgt agc act agg 2256
Ile Glu Pro Arg Ser Phe Ser Gln Asn Ser Arg His Arg Ser Thr Arg
            740                 745                 750

caa aag caa ttt aat gcc acc aca att cca gaa aat gac ata gag aag 2304
Gln Lys Gln Phe Asn Ala Thr Thr Ile Pro Glu Asn Asp Ile Glu Lys
        755                 760                 765

act gac cct tgg ttt gca cac aga aca cct atg cct aaa ata caa aat 2352
Thr Asp Pro Trp Phe Ala His Arg Thr Pro Met Pro Lys Ile Gln Asn
    770                 775                 780

gtc tcc tct agt gat ttg ttg atg ctc ttg cga cag agt cct act cca 2400
Val Ser Ser Ser Asp Leu Leu Met Leu Leu Arg Gln Ser Pro Thr Pro
785                 790                 795                 800

cat ggg cta tcc tta tct gat ctc caa gaa gcc aaa tat gag act ttt 2448
His Gly Leu Ser Leu Ser Asp Leu Gln Glu Ala Lys Tyr Glu Thr Phe
                805                 810                 815

tct gat gat cca tca cct gga gca ata gac agt aat aac agc ctg tct 2496
Ser Asp Asp Pro Ser Pro Gly Ala Ile Asp Ser Asn Asn Ser Leu Ser
            820                 825                 830

gaa atg aca cac ttc agg cca cag ctc cat cac agt ggg gac atg gta 2544
Glu Met Thr His Phe Arg Pro Gln Leu His His Ser Gly Asp Met Val
        835                 840                 845

ttt acc cct gag tca ggc ctc caa tta aga tta aat gag aaa ctg ggg 2592
Phe Thr Pro Glu Ser Gly Leu Gln Leu Arg Leu Asn Glu Lys Leu Gly
    850                 855                 860

aca act gca gca aca gag ttg aag aaa ctt gat ttc aaa gtt tct agt 2640
Thr Thr Ala Ala Thr Glu Leu Lys Lys Leu Asp Phe Lys Val Ser Ser
865                 870                 875                 880

aca tca aat aat ctg att tca aca att cca tca gac aat ttg gca gca 2688
Thr Ser Asn Asn Leu Ile Ser Thr Ile Pro Ser Asp Asn Leu Ala Ala
                885                 890                 895

ggt act gat aat aca agt tcc tta gga ccc cca agt atg cca gtt cat 2736
Gly Thr Asp Asn Thr Ser Ser Leu Gly Pro Pro Ser Met Pro Val His
            900                 905                 910

tat gat agt caa tta gat acc act cta ttt ggc aaa aag tca tct ccc 2784
Tyr Asp Ser Gln Leu Asp Thr Thr Leu Phe Gly Lys Lys Ser Ser Pro
        915                 920                 925

ctt act gag tct ggt gga cct ctg agc ttg agt gaa gaa aat aat gat 2832
Leu Thr Glu Ser Gly Gly Pro Leu Ser Leu Ser Glu Glu Asn Asn Asp
    930                 935                 940

tca aag ttg tta gaa tca ggt tta atg aat agc caa gaa agt tca tgg 2880
Ser Lys Leu Leu Glu Ser Gly Leu Met Asn Ser Gln Glu Ser Ser Trp
945                 950                 955                 960

gga aaa aat gta tcg tca aca gag agt ggt agg tta ttt aaa ggg aaa 2928
Gly Lys Asn Val Ser Ser Thr Glu Ser Gly Arg Leu Phe Lys Gly Lys
                965                 970                 975

aga gct cat gga cct gct ttg ttg act aaa gat aat gcc tta ttc aaa 2976
Arg Ala His Gly Pro Ala Leu Leu Thr Lys Asp Asn Ala Leu Phe Lys
            980                 985                 990

gtt agc atc tct ttg tta aag aca aac aaa act tcc aat aat tca gca 3024
Val Ser Ile Ser Leu Leu Lys Thr Asn Lys Thr Ser Asn Asn Ser Ala
        995                 1000                1005

act aat aga aag act cac att gat ggc cca tca tta tta att gag 3069
Thr Asn Arg Lys Thr His Ile Asp Gly Pro Ser Leu Leu Ile Glu
    1010                1015                1020

aat agt cca tca gtc tgg caa aat ata tta gaa agt gac act gag 3114
Asn Ser Pro Ser Val Trp Gln Asn Ile Leu Glu Ser Asp Thr Glu
    1025                1030                1035

ttt aaa aaa gtg aca cct ttg att cat gac aga atg ctt atg gac 3159
Phe Lys Lys Val Thr Pro Leu Ile His Asp Arg Met Leu Met Asp
    1040                1045                1050

aaa aat gct aca gct ttg agg cta aat cat atg tca aat aaa act 3204
Lys Asn Ala Thr Ala Leu Arg Leu Asn His Met Ser Asn Lys Thr
    1055                1060                1065

act tca tca aaa aac atg gaa atg gtc caa cag aaa aaa gag ggc 3249
Thr Ser Ser Lys Asn Met Glu Met Val Gln Gln Lys Lys Glu Gly
    1070                1075                1080

ccc att cca cca gat gca caa aat cca gat atg tcg ttc ttt aag 3294
Pro Ile Pro Pro Asp Ala Gln Asn Pro Asp Met Ser Phe Phe Lys
    1085                1090                1095

atg cta ttc ttg cca gaa tca gca agg tgg ata caa agg act cat 3339
Met Leu Phe Leu Pro Glu Ser Ala Arg Trp Ile Gln Arg Thr His
    1100                1105                1110

gga aag aac tct ctg aac tct ggg caa ggc ccc agt cca aag caa 3384
Gly Lys Asn Ser Leu Asn Ser Gly Gln Gly Pro Ser Pro Lys Gln
    1115                1120                1125

tta gta tcc tta gga cca gaa aaa tct gtg gaa ggt cag aat ttc 3429
Leu Val Ser Leu Gly Pro Glu Lys Ser Val Glu Gly Gln Asn Phe
    1130                1135                1140

ttg tct gag aaa aac aaa gtg gta gta gga aag ggt gaa ttt aca 3474
Leu Ser Glu Lys Asn Lys Val Val Val Gly Lys Gly Glu Phe Thr
    1145                1150                1155

aag gac gta gga ctc aaa gag atg gtt ttt cca agc agc aga aac 3519
Lys Asp Val Gly Leu Lys Glu Met Val Phe Pro Ser Ser Arg Asn
    1160                1165                1170

cta ttt ctt act aac ttg gat aat tta cat gaa aat aat aca cac 3564
Leu Phe Leu Thr Asn Leu Asp Asn Leu His Glu Asn Asn Thr His
    1175                1180                1185

aat caa gaa aaa aaa att cag gaa gaa ata gaa aag aag gaa aca 3609
Asn Gln Glu Lys Lys Ile Gln Glu Glu Ile Glu Lys Lys Glu Thr
    1190                1195                1200

tta atc caa gag aat gta gtt ttg cct cag ata cat aca gtg act 3654
Leu Ile Gln Glu Asn Val Val Leu Pro Gln Ile His Thr Val Thr
    1205                1210                1215

ggc act aag aat ttc atg aag aac ctt ttc tta ctg agc act agg 3699
Gly Thr Lys Asn Phe Met Lys Asn Leu Phe Leu Leu Ser Thr Arg
    1220                1225                1230

caa aat gta gaa ggt tca tat gac ggg gca tat gct cca gta ctt 3744
Gln Asn Val Glu Gly Ser Tyr Asp Gly Ala Tyr Ala Pro Val Leu
    1235                1240                1245

caa gat ttt agg tca tta aat gat tca aca aat aga aca aag aaa 3789
Gln Asp Phe Arg Ser Leu Asn Asp Ser Thr Asn Arg Thr Lys Lys
    1250                1255                1260

cac aca gct cat ttc tca aaa aaa ggg gag gaa gaa aac ttg gaa 3834

His Thr Ala His Phe Ser Lys Lys Gly Glu Glu Glu Asn Leu Glu
    1265                1270                1275

ggc ttg gga aat caa acc aag caa att gta gag aaa tat gca tgc      3879
Gly Leu Gly Asn Gln Thr Lys Gln Ile Val Glu Lys Tyr Ala Cys
    1280                1285                1290

acc aca agg ata tct cct aat aca agc cag cag aat ttt gtc acg      3924
Thr Thr Arg Ile Ser Pro Asn Thr Ser Gln Gln Asn Phe Val Thr
    1295                1300                1305

caa cgt agt aag aga gct ttg aaa caa ttc aga ctc cca cta gaa      3969
Gln Arg Ser Lys Arg Ala Leu Lys Gln Phe Arg Leu Pro Leu Glu
    1310                1315                1320

gaa aca gaa ctt gaa aaa agg ata att gtg gat gac acc tca acc      4014
Glu Thr Glu Leu Glu Lys Arg Ile Ile Val Asp Asp Thr Ser Thr
    1325                1330                1335

cag tgg tcc aaa aac atg aaa cat ttg acc ccg agc acc ctc aca      4059
Gln Trp Ser Lys Asn Met Lys His Leu Thr Pro Ser Thr Leu Thr
    1340                1345                1350

cag ata gac tac aat gag aag gag aaa ggg gcc att act cag tct      4104
Gln Ile Asp Tyr Asn Glu Lys Glu Lys Gly Ala Ile Thr Gln Ser
    1355                1360                1365

ccc tta tca gat tgc ctt acg agg agt cat agc atc cct caa gca      4149
Pro Leu Ser Asp Cys Leu Thr Arg Ser His Ser Ile Pro Gln Ala
    1370                1375                1380

aat aga tct cca tta ccc att gca aag gta tca tca ttt cca tct      4194
Asn Arg Ser Pro Leu Pro Ile Ala Lys Val Ser Ser Phe Pro Ser
    1385                1390                1395

att aga cct ata tat ctg acc agg gtc cta ttc caa gac aac tct      4239
Ile Arg Pro Ile Tyr Leu Thr Arg Val Leu Phe Gln Asp Asn Ser
    1400                1405                1410

tct cat ctt cca gca gca tct tat aga aag aaa gat tct ggg gtc      4284
Ser His Leu Pro Ala Ala Ser Tyr Arg Lys Lys Asp Ser Gly Val
    1415                1420                1425

caa gaa agc agt cat ttc tta caa gga gcc aaa aaa aat aac ctt      4329
Gln Glu Ser Ser His Phe Leu Gln Gly Ala Lys Lys Asn Asn Leu
    1430                1435                1440

tct tta gcc att cta acc ttg gag atg act ggt gat caa aga gag      4374
Ser Leu Ala Ile Leu Thr Leu Glu Met Thr Gly Asp Gln Arg Glu
    1445                1450                1455

gtt ggc tcc ctg ggg aca agt gcc aca aat tca gtc aca tac aag      4419
Val Gly Ser Leu Gly Thr Ser Ala Thr Asn Ser Val Thr Tyr Lys
    1460                1465                1470

aaa gtt gag aac act gtt ctc ccg aaa cca gac ttg ccc aaa aca      4464
Lys Val Glu Asn Thr Val Leu Pro Lys Pro Asp Leu Pro Lys Thr
    1475                1480                1485

tct ggc aaa gtt gaa ttg ctt cca aaa gtt cac att tat cag aag      4509
Ser Gly Lys Val Glu Leu Leu Pro Lys Val His Ile Tyr Gln Lys
    1490                1495                1500

gac cta ttc cct acg gaa act agc aat ggg tct cct ggc cat ctg      4554
Asp Leu Phe Pro Thr Glu Thr Ser Asn Gly Ser Pro Gly His Leu
    1505                1510                1515

gat ctc gtg gaa ggg agc ctt ctt cag gga aca gag gga gcg att      4599
Asp Leu Val Glu Gly Ser Leu Leu Gln Gly Thr Glu Gly Ala Ile

1520                    1525                    1530

aag tgg aat gaa gca aac aga cct gga aaa gtt ccc ttt ctg aga        4644
Lys Trp Asn Glu Ala Asn Arg Pro Gly Lys Val Pro Phe Leu Arg
    1535                    1540                    1545

gta gca aca gaa agc tct gca aag act ccc tcc aag cta ttg gat        4689
Val Ala Thr Glu Ser Ser Ala Lys Thr Pro Ser Lys Leu Leu Asp
    1550                    1555                    1560

cct ctt gct tgg gat aac cac tat ggt act cag ata cca aaa gaa        4734
Pro Leu Ala Trp Asp Asn His Tyr Gly Thr Gln Ile Pro Lys Glu
    1565                    1570                    1575

gag tgg aaa tcc caa gag aag tca cca gaa aaa aca gct ttt aag        4779
Glu Trp Lys Ser Gln Glu Lys Ser Pro Glu Lys Thr Ala Phe Lys
    1580                    1585                    1590

aaa aag gat acc att ttg tcc ctg aac gct tgt gaa agc aat cat        4824
Lys Lys Asp Thr Ile Leu Ser Leu Asn Ala Cys Glu Ser Asn His
    1595                    1600                    1605

gca ata gca gca ata aat gag gga caa aat aag ccc gaa ata gaa        4869
Ala Ile Ala Ala Ile Asn Glu Gly Gln Asn Lys Pro Glu Ile Glu
    1610                    1615                    1620

gtc acc tgg gca aag caa ggt agg act gaa agg ctg tgc tct caa        4914
Val Thr Trp Ala Lys Gln Gly Arg Thr Glu Arg Leu Cys Ser Gln
    1625                    1630                    1635

aac cca cca gtc ttg aaa cgc cat caa cgg gaa ata act cgt act        4959
Asn Pro Pro Val Leu Lys Arg His Gln Arg Glu Ile Thr Arg Thr
    1640                    1645                    1650

act ctt cag tca gat caa gag gaa att gac tat gat gat acc ata        5004
Thr Leu Gln Ser Asp Gln Glu Glu Ile Asp Tyr Asp Asp Thr Ile
    1655                    1660                    1665

tca gtt gaa atg aag aag gaa gat ttt gac att tat gat gag gat        5049
Ser Val Glu Met Lys Lys Glu Asp Phe Asp Ile Tyr Asp Glu Asp
    1670                    1675                    1680

gaa aat cag agc ccc cgc agc ttt caa aag aaa aca cga cac tat        5094
Glu Asn Gln Ser Pro Arg Ser Phe Gln Lys Lys Thr Arg His Tyr
    1685                    1690                    1695

ttt att gct gca gtg gag agg ctc tgg gat tat ggg atg agt agc        5139
Phe Ile Ala Ala Val Glu Arg Leu Trp Asp Tyr Gly Met Ser Ser
    1700                    1705                    1710

tcc cca cat gtt cta aga aac agg gct cag agt ggc agt gtc cct        5184
Ser Pro His Val Leu Arg Asn Arg Ala Gln Ser Gly Ser Val Pro
    1715                    1720                    1725

cag ttc aag aaa gtt gtt ttc cag gaa ttt act gat ggc tcc ttt        5229
Gln Phe Lys Lys Val Val Phe Gln Glu Phe Thr Asp Gly Ser Phe
    1730                    1735                    1740

act cag ccc tta tac cgt gga gaa cta aat gaa cat ttg gga ctc        5274
Thr Gln Pro Leu Tyr Arg Gly Glu Leu Asn Glu His Leu Gly Leu
    1745                    1750                    1755

ctg ggg cca tat ata aga gca gaa gtt gaa gat aat atc atg gta        5319
Leu Gly Pro Tyr Ile Arg Ala Glu Val Glu Asp Asn Ile Met Val
    1760                    1765                    1770

act ttc aga aat cag gcc tct cgt ccc tat tcc ttc tat tct agc        5364
Thr Phe Arg Asn Gln Ala Ser Arg Pro Tyr Ser Phe Tyr Ser Ser
    1775                    1780                    1785

ctt att tct tat gag gaa gat cag agg caa gga gca gaa cct aga 5409
Leu Ile Ser Tyr Glu Glu Asp Gln Arg Gln Gly Ala Glu Pro Arg
    1790                1795                1800

aaa aac ttt gtc aag cct aat gaa acc aaa act tac ttt tgg aaa 5454
Lys Asn Phe Val Lys Pro Asn Glu Thr Lys Thr Tyr Phe Trp Lys
    1805                1810                1815

gtg caa cat cat atg gca ccc act aaa gat gag ttt gac tgc aaa 5499
Val Gln His His Met Ala Pro Thr Lys Asp Glu Phe Asp Cys Lys
    1820                1825                1830

gcc tgg gct tat ttc tct gat gtt gac ctg gaa aaa gat gtg cac 5544
Ala Trp Ala Tyr Phe Ser Asp Val Asp Leu Glu Lys Asp Val His
    1835                1840                1845

tca ggc ctg att gga ccc ctt ctg gtc tgc cac act aac aca ctg 5589
Ser Gly Leu Ile Gly Pro Leu Leu Val Cys His Thr Asn Thr Leu
    1850                1855                1860

aac cct gct cat ggg aga caa gtg aca gta cag gaa ttt gct ctg 5634
Asn Pro Ala His Gly Arg Gln Val Thr Val Gln Glu Phe Ala Leu
    1865                1870                1875

ttt ttc acc atc ttt gat gag acc aaa agc tgg tac ttc act gaa 5679
Phe Phe Thr Ile Phe Asp Glu Thr Lys Ser Trp Tyr Phe Thr Glu
    1880                1885                1890

aat atg gaa aga aac tgc agg gct ccc tgc aat atc cag atg gaa 5724
Asn Met Glu Arg Asn Cys Arg Ala Pro Cys Asn Ile Gln Met Glu
    1895                1900                1905

gat ccc act ttt aaa gag aat tat cgc ttc cat gca atc aat ggc 5769
Asp Pro Thr Phe Lys Glu Asn Tyr Arg Phe His Ala Ile Asn Gly
    1910                1915                1920

tac ata atg gat aca cta cct ggc tta gta atg gct cag gat caa 5814
Tyr Ile Met Asp Thr Leu Pro Gly Leu Val Met Ala Gln Asp Gln
    1925                1930                1935

agg att cga tgg tat ctg ctc agc atg ggc agc aat gaa aac atc 5859
Arg Ile Arg Trp Tyr Leu Leu Ser Met Gly Ser Asn Glu Asn Ile
    1940                1945                1950

cat tct att cat ttc agt gga cat gtg ttc act gta cga aaa aaa 5904
His Ser Ile His Phe Ser Gly His Val Phe Thr Val Arg Lys Lys
    1955                1960                1965

gag gag tat aaa atg gca ctg tac aat ctc tat cca ggt gtt ttt 5949
Glu Glu Tyr Lys Met Ala Leu Tyr Asn Leu Tyr Pro Gly Val Phe
    1970                1975                1980

gag aca gtg gaa atg tta cca tcc aaa gct gga att tgg cgg gtg 5994
Glu Thr Val Glu Met Leu Pro Ser Lys Ala Gly Ile Trp Arg Val
    1985                1990                1995

gaa tgc ctt att ggc gag cat cta cat gct ggg atg agc aca ctt 6039
Glu Cys Leu Ile Gly Glu His Leu His Ala Gly Met Ser Thr Leu
    2000                2005                2010

ttt ctg gtg tac agc aat aag tgt cag act ccc ctg gga atg gct 6084
Phe Leu Val Tyr Ser Asn Lys Cys Gln Thr Pro Leu Gly Met Ala
    2015                2020                2025

tct gga cac att aga gat ttt cag att aca gct tca gga caa tat 6129
Ser Gly His Ile Arg Asp Phe Gln Ile Thr Ala Ser Gly Gln Tyr
    2030                2035                2040

gga cag tgg gcc cca aag ctg gcc aga ctt cat tat tcc gga tca 6174
Gly Gln Trp Ala Pro Lys Leu Ala Arg Leu His Tyr Ser Gly Ser
    2045                2050                2055

atc aat gcc tgg agc acc aag gag ccc ttt tct tgg atc aag gtg 6219
Ile Asn Ala Trp Ser Thr Lys Glu Pro Phe Ser Trp Ile Lys Val
    2060                2065                2070

gat ctg ttg gca cca atg att att cac ggc atc aag acc cag ggt 6264
Asp Leu Leu Ala Pro Met Ile Ile His Gly Ile Lys Thr Gln Gly
    2075                2080                2085

gcc cgt cag aag ttc tcc agc ctc tac atc tct cag ttt atc atc 6309
Ala Arg Gln Lys Phe Ser Ser Leu Tyr Ile Ser Gln Phe Ile Ile
    2090                2095                2100

atg tat agt ctt gat ggg aag aag tgg cag act tat cga gga aat 6354
Met Tyr Ser Leu Asp Gly Lys Lys Trp Gln Thr Tyr Arg Gly Asn
    2105                2110                2115

tcc act gga acc tta atg gtc ttc ttt ggc aat gtg gat tca tct 6399
Ser Thr Gly Thr Leu Met Val Phe Phe Gly Asn Val Asp Ser Ser
    2120                2125                2130

ggg ata aaa cac aat att ttt aac cct cca att att gct cga tac 6444
Gly Ile Lys His Asn Ile Phe Asn Pro Pro Ile Ile Ala Arg Tyr
    2135                2140                2145

atc cgt ttg cac cca act cat tat agc att cgc agc act ctt cgc 6489
Ile Arg Leu His Pro Thr His Tyr Ser Ile Arg Ser Thr Leu Arg
    2150                2155                2160

atg gag ttg atg ggc tgt gat tta aat agt tgc agc atg cca ttg 6534
Met Glu Leu Met Gly Cys Asp Leu Asn Ser Cys Ser Met Pro Leu
    2165                2170                2175

gga atg gag agt aaa gca ata tca gat gca cag att act gct tca 6579
Gly Met Glu Ser Lys Ala Ile Ser Asp Ala Gln Ile Thr Ala Ser
    2180                2185                2190

tcc tac ttt acc aat atg ttt gcc acc tgg tct cct tca aaa gct 6624
Ser Tyr Phe Thr Asn Met Phe Ala Thr Trp Ser Pro Ser Lys Ala
    2195                2200                2205

cga ctt cac ctc caa ggg agg agt aat gcc tgg aga cct cag gtg 6669
Arg Leu His Leu Gln Gly Arg Ser Asn Ala Trp Arg Pro Gln Val
    2210                2215                2220

aat aat cca aaa gag tgg ctg caa gtg gac ttc cag aag aca atg 6714
Asn Asn Pro Lys Glu Trp Leu Gln Val Asp Phe Gln Lys Thr Met
    2225                2230                2235

aaa gtc aca gga gta act act cag gga gta aaa tct ctg ctt acc 6759
Lys Val Thr Gly Val Thr Thr Gln Gly Val Lys Ser Leu Leu Thr
    2240                2245                2250

agc atg tat gtg aag gag ttc ctc atc tcc agc agt caa gat ggc 6804
Ser Met Tyr Val Lys Glu Phe Leu Ile Ser Ser Ser Gln Asp Gly
    2255                2260                2265

cat cag tgg act ctc ttt ttt cag aat ggc aaa gta aag gtt ttt 6849
His Gln Trp Thr Leu Phe Phe Gln Asn Gly Lys Val Lys Val Phe
    2270                2275                2280

cag gga aat caa gac tcc ttc aca cct gtg gtg aac tct cta gac 6894
Gln Gly Asn Gln Asp Ser Phe Thr Pro Val Val Asn Ser Leu Asp
    2285                2290                2295

cca ccg tta ctg act cgc tac ctt cga att cac ccc cag agt tgg 6939

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Arg Ser Thr Arg
            740                 745                 750

Gln Lys Gln Phe Asn Ala Thr Thr Ile Pro Glu Asn Asp Ile Glu Lys
755 760 765

Thr Asp Pro Trp Phe Ala His Arg Thr Pro Met Pro Lys Ile Gln Asn
770 775 780

Val Ser Ser Ser Asp Leu Leu Met Leu Leu Arg Gln Ser Pro Thr Pro
785 790 795 800

His Gly Leu Ser Leu Ser Asp Leu Gln Glu Ala Lys Tyr Glu Thr Phe
805 810 815

Ser Asp Asp Pro Ser Pro Gly Ala Ile Asp Ser Asn Asn Ser Leu Ser
820 825 830

Glu Met Thr His Phe Arg Pro Gln Leu His His Ser Gly Asp Met Val
835 840 845

Phe Thr Pro Glu Ser Gly Leu Gln Leu Arg Leu Asn Glu Lys Leu Gly
850 855 860

Thr Thr Ala Ala Thr Glu Leu Lys Lys Leu Asp Phe Lys Val Ser Ser
865 870 875 880

Thr Ser Asn Asn Leu Ile Ser Thr Ile Pro Ser Asp Asn Leu Ala Ala
885 890 895

Gly Thr Asp Asn Thr Ser Ser Leu Gly Pro Pro Ser Met Pro Val His
900 905 910

Tyr Asp Ser Gln Leu Asp Thr Thr Leu Phe Gly Lys Lys Ser Ser Pro
915 920 925

Leu Thr Glu Ser Gly Gly Pro Leu Ser Leu Ser Glu Glu Asn Asn Asp
930 935 940

Ser Lys Leu Leu Glu Ser Gly Leu Met Asn Ser Gln Glu Ser Ser Trp
945 950 955 960

Gly Lys Asn Val Ser Ser Thr Glu Ser Gly Arg Leu Phe Lys Gly Lys
965 970 975

Arg Ala His Gly Pro Ala Leu Leu Thr Lys Asp Asn Ala Leu Phe Lys
980 985 990

Val Ser Ile Ser Leu Leu Lys Thr Asn Lys Thr Ser Asn Asn Ser Ala
995 1000 1005

Thr Asn Arg Lys Thr His Ile Asp Gly Pro Ser Leu Leu Ile Glu
1010 1015 1020

Asn Ser Pro Ser Val Trp Gln Asn Ile Leu Glu Ser Asp Thr Glu

1025                 1030                 1035

Phe Lys  Lys Val Thr Pro Leu  Ile His Asp Arg Met  Leu Met Asp
    1040                 1045                 1050

Lys Asn  Ala Thr Ala Leu Arg  Leu Asn His Met Ser  Asn Lys Thr
    1055                 1060                 1065

Thr Ser  Ser Lys Asn Met Glu  Met Val Gln Gln Lys  Lys Glu Gly
    1070                 1075                 1080

Pro Ile  Pro Pro Asp Ala Gln  Asn Pro Asp Met Ser  Phe Phe Lys
    1085                 1090                 1095

Met Leu  Phe Leu Pro Glu Ser  Ala Arg Trp Ile Gln  Arg Thr His
    1100                 1105                 1110

Gly Lys  Asn Ser Leu Asn Ser  Gly Gln Gly Pro Ser  Pro Lys Gln
    1115                 1120                 1125

Leu Val  Ser Leu Gly Pro Glu  Lys Ser Val Glu Gly  Gln Asn Phe
    1130                 1135                 1140

Leu Ser  Glu Lys Asn Lys Val  Val Val Gly Lys Gly  Glu Phe Thr
    1145                 1150                 1155

Lys Asp  Val Gly Leu Lys Glu  Met Val Phe Pro Ser  Ser Arg Asn
    1160                 1165                 1170

Leu Phe  Leu Thr Asn Leu Asp  Asn Leu His Glu Asn  Asn Thr His
    1175                 1180                 1185

Asn Gln  Glu Lys Lys Ile Gln  Glu Glu Ile Glu Lys  Lys Glu Thr
    1190                 1195                 1200

Leu Ile  Gln Glu Asn Val Val  Leu Pro Gln Ile His  Thr Val Thr
    1205                 1210                 1215

Gly Thr  Lys Asn Phe Met Lys  Asn Leu Phe Leu Leu  Ser Thr Arg
    1220                 1225                 1230

Gln Asn  Val Glu Gly Ser Tyr  Asp Gly Ala Tyr Ala  Pro Val Leu
    1235                 1240                 1245

Gln Asp  Phe Arg Ser Leu Asn  Asp Ser Thr Asn Arg  Thr Lys Lys
    1250                 1255                 1260

His Thr  Ala His Phe Ser Lys  Lys Gly Glu Glu Glu  Asn Leu Glu
    1265                 1270                 1275

Gly Leu  Gly Asn Gln Thr Lys  Gln Ile Val Glu Lys  Tyr Ala Cys
    1280                 1285                 1290

Thr Thr Arg Ile Ser Pro Asn Thr Ser Gln Gln Asn Phe Val Thr
1295 1300 1305

Gln Arg Ser Lys Arg Ala Leu Lys Gln Phe Arg Leu Pro Leu Glu
1310 1315 1320

Glu Thr Glu Leu Glu Lys Arg Ile Ile Val Asp Asp Thr Ser Thr
1325 1330 1335

Gln Trp Ser Lys Asn Met Lys His Leu Thr Pro Ser Thr Leu Thr
1340 1345 1350

Gln Ile Asp Tyr Asn Glu Lys Glu Lys Gly Ala Ile Thr Gln Ser
1355 1360 1365

Pro Leu Ser Asp Cys Leu Thr Arg Ser His Ser Ile Pro Gln Ala
1370 1375 1380

Asn Arg Ser Pro Leu Pro Ile Ala Lys Val Ser Ser Phe Pro Ser
1385 1390 1395

Ile Arg Pro Ile Tyr Leu Thr Arg Val Leu Phe Gln Asp Asn Ser
1400 1405 1410

Ser His Leu Pro Ala Ala Ser Tyr Arg Lys Lys Asp Ser Gly Val
1415 1420 1425

Gln Glu Ser Ser His Phe Leu Gln Gly Ala Lys Lys Asn Asn Leu
1430 1435 1440

Ser Leu Ala Ile Leu Thr Leu Glu Met Thr Gly Asp Gln Arg Glu
1445 1450 1455

Val Gly Ser Leu Gly Thr Ser Ala Thr Asn Ser Val Thr Tyr Lys
1460 1465 1470

Lys Val Glu Asn Thr Val Leu Pro Lys Pro Asp Leu Pro Lys Thr
1475 1480 1485

Ser Gly Lys Val Glu Leu Leu Pro Lys Val His Ile Tyr Gln Lys
1490 1495 1500

Asp Leu Phe Pro Thr Glu Thr Ser Asn Gly Ser Pro Gly His Leu
1505 1510 1515

Asp Leu Val Glu Gly Ser Leu Leu Gln Gly Thr Glu Gly Ala Ile
1520 1525 1530

Lys Trp Asn Glu Ala Asn Arg Pro Gly Lys Val Pro Phe Leu Arg
1535 1540 1545

Val Ala Thr Glu Ser Ser Ala Lys Thr Pro Ser Lys Leu Leu Asp
    1550                    1555                    1560

Pro Leu Ala Trp Asp Asn His Tyr Gly Thr Gln Ile Pro Lys Glu
    1565                    1570                    1575

Glu Trp Lys Ser Gln Glu Lys Ser Pro Glu Lys Thr Ala Phe Lys
    1580                    1585                    1590

Lys Lys Asp Thr Ile Leu Ser Leu Asn Ala Cys Glu Ser Asn His
    1595                    1600                    1605

Ala Ile Ala Ala Ile Asn Glu Gly Gln Asn Lys Pro Glu Ile Glu
    1610                    1615                    1620

Val Thr Trp Ala Lys Gln Gly Arg Thr Glu Arg Leu Cys Ser Gln
    1625                    1630                    1635

Asn Pro Pro Val Leu Lys Arg His Gln Arg Glu Ile Thr Arg Thr
    1640                    1645                    1650

Thr Leu Gln Ser Asp Gln Glu Glu Ile Asp Tyr Asp Asp Thr Ile
    1655                    1660                    1665

Ser Val Glu Met Lys Lys Glu Asp Phe Asp Ile Tyr Asp Glu Asp
    1670                    1675                    1680

Glu Asn Gln Ser Pro Arg Ser Phe Gln Lys Lys Thr Arg His Tyr
    1685                    1690                    1695

Phe Ile Ala Ala Val Glu Arg Leu Trp Asp Tyr Gly Met Ser Ser
    1700                    1705                    1710

Ser Pro His Val Leu Arg Asn Arg Ala Gln Ser Gly Ser Val Pro
    1715                    1720                    1725

Gln Phe Lys Lys Val Val Phe Gln Glu Phe Thr Asp Gly Ser Phe
    1730                    1735                    1740

Thr Gln Pro Leu Tyr Arg Gly Glu Leu Asn Glu His Leu Gly Leu
    1745                    1750                    1755

Leu Gly Pro Tyr Ile Arg Ala Glu Val Glu Asp Asn Ile Met Val
    1760                    1765                    1770

Thr Phe Arg Asn Gln Ala Ser Arg Pro Tyr Ser Phe Tyr Ser Ser
    1775                    1780                    1785

Leu Ile Ser Tyr Glu Glu Asp Gln Arg Gln Gly Ala Glu Pro Arg
    1790                    1795                    1800

Lys Asn Phe Val Lys Pro Asn Glu Thr Lys Thr Tyr Phe Trp Lys
    1805                1810                1815

Val Gln His His Met Ala Pro Thr Lys Asp Glu Phe Asp Cys Lys
    1820                1825                1830

Ala Trp Ala Tyr Phe Ser Asp Val Asp Leu Glu Lys Asp Val His
    1835                1840                1845

Ser Gly Leu Ile Gly Pro Leu Leu Val Cys His Thr Asn Thr Leu
    1850                1855                1860

Asn Pro Ala His Gly Arg Gln Val Thr Val Gln Glu Phe Ala Leu
    1865                1870                1875

Phe Phe Thr Ile Phe Asp Glu Thr Lys Ser Trp Tyr Phe Thr Glu
    1880                1885                1890

Asn Met Glu Arg Asn Cys Arg Ala Pro Cys Asn Ile Gln Met Glu
    1895                1900                1905

Asp Pro Thr Phe Lys Glu Asn Tyr Arg Phe His Ala Ile Asn Gly
    1910                1915                1920

Tyr Ile Met Asp Thr Leu Pro Gly Leu Val Met Ala Gln Asp Gln
    1925                1930                1935

Arg Ile Arg Trp Tyr Leu Leu Ser Met Gly Ser Asn Glu Asn Ile
    1940                1945                1950

His Ser Ile His Phe Ser Gly His Val Phe Thr Val Arg Lys Lys
    1955                1960                1965

Glu Glu Tyr Lys Met Ala Leu Tyr Asn Leu Tyr Pro Gly Val Phe
    1970                1975                1980

Glu Thr Val Glu Met Leu Pro Ser Lys Ala Gly Ile Trp Arg Val
    1985                1990                1995

Glu Cys Leu Ile Gly Glu His Leu His Ala Gly Met Ser Thr Leu
    2000                2005                2010

Phe Leu Val Tyr Ser Asn Lys Cys Gln Thr Pro Leu Gly Met Ala
    2015                2020                2025

Ser Gly His Ile Arg Asp Phe Gln Ile Thr Ala Ser Gly Gln Tyr
    2030                2035                2040

Gly Gln Trp Ala Pro Lys Leu Ala Arg Leu His Tyr Ser Gly Ser
    2045                2050                2055

Ile Asn Ala Trp Ser Thr Lys Glu Pro Phe Ser Trp Ile Lys Val

2060                 2065                 2070

Asp Leu  Leu Ala Pro Met Ile  Ile His Gly Ile Lys  Thr Gln Gly
    2075                 2080                 2085

Ala Arg  Gln Lys Phe Ser Ser  Leu Tyr Ile Ser Gln  Phe Ile Ile
    2090                 2095                 2100

Met Tyr  Ser Leu Asp Gly Lys  Lys Trp Gln Thr Tyr  Arg Gly Asn
    2105                 2110                 2115

Ser Thr  Gly Thr Leu Met Val  Phe Phe Gly Asn Val  Asp Ser Ser
    2120                 2125                 2130

Gly Ile  Lys His Asn Ile Phe  Asn Pro Pro Ile Ile  Ala Arg Tyr
    2135                 2140                 2145

Ile Arg  Leu His Pro Thr His  Tyr Ser Ile Arg Ser  Thr Leu Arg
    2150                 2155                 2160

Met Glu  Leu Met Gly Cys Asp  Leu Asn Ser Cys Ser  Met Pro Leu
    2165                 2170                 2175

Gly Met  Glu Ser Lys Ala Ile  Ser Asp Ala Gln Ile  Thr Ala Ser
    2180                 2185                 2190

Ser Tyr  Phe Thr Asn Met Phe  Ala Thr Trp Ser Pro  Ser Lys Ala
    2195                 2200                 2205

Arg Leu  His Leu Gln Gly Arg  Ser Asn Ala Trp Arg  Pro Gln Val
    2210                 2215                 2220

Asn Asn  Pro Lys Glu Trp Leu  Gln Val Asp Phe Gln  Lys Thr Met
    2225                 2230                 2235

Lys Val  Thr Gly Val Thr Thr  Gln Gly Val Lys Ser  Leu Leu Thr
    2240                 2245                 2250

Ser Met  Tyr Val Lys Glu Phe  Leu Ile Ser Ser Ser  Gln Asp Gly
    2255                 2260                 2265

His Gln  Trp Thr Leu Phe Phe  Gln Asn Gly Lys Val  Lys Val Phe
    2270                 2275                 2280

Gln Gly  Asn Gln Asp Ser Phe  Thr Pro Val Val Asn  Ser Leu Asp
    2285                 2290                 2295

Pro Pro  Leu Leu Thr Arg Tyr  Leu Arg Ile His Pro  Gln Ser Trp
    2300                 2305                 2310

Val His  Gln Ile Ala Leu Arg  Met Glu Val Leu Gly  Cys Glu Ala
    2315                 2320                 2325

tat gtg aat ggg aca atg cca gat ata aca gtt tgt gcc cat gac cac 672
Tyr Val Asn Gly Thr Met Pro Asp Ile Thr Val Cys Ala His Asp His
210 215 220

atc agc tgg cat ctg ctg gga atg agc tcg ggg cca gaa tta ttc tcc 720
Ile Ser Trp His Leu Leu Gly Met Ser Ser Gly Pro Glu Leu Phe Ser
225 230 235 240

att cat ttc aac ggc cag gtc ctg gag cag aac cat cat aag gtc tca 768
Ile His Phe Asn Gly Gln Val Leu Glu Gln Asn His His Lys Val Ser
245 250 255

gcc atc acc ctt gtc agt gct aca tcc act acc gca aat atg act gtg 816
Ala Ile Thr Leu Val Ser Ala Thr Ser Thr Thr Ala Asn Met Thr Val
260 265 270

ggc cca gag gga aag tgg atc ata tct tct ctc acc cca aaa cat ttg 864
Gly Pro Glu Gly Lys Trp Ile Ile Ser Ser Leu Thr Pro Lys His Leu
275 280 285

caa gct ggg atg cag gct tac att gac att aaa aac tgc cca aag aaa 912
Gln Ala Gly Met Gln Ala Tyr Ile Asp Ile Lys Asn Cys Pro Lys Lys
290 295 300

acc agg aat ctt aag aaa ata act cgt gag cag agg cgg cac atg aag 960
Thr Arg Asn Leu Lys Lys Ile Thr Arg Glu Gln Arg Arg His Met Lys
305 310 315 320

agg tgg gaa tac ttc att gct gca gag gaa gtc att tgg gac tat gca 1008
Arg Trp Glu Tyr Phe Ile Ala Ala Glu Glu Val Ile Trp Asp Tyr Ala
325 330 335

cct gta ata cca gcg aat atg gac aaa aaa tac agg tct cag cat ttg 1056
Pro Val Ile Pro Ala Asn Met Asp Lys Lys Tyr Arg Ser Gln His Leu
340 345 350

gat aat ttc tca aac caa att gga aaa cat tat aag aaa gtt atg tac 1104
Asp Asn Phe Ser Asn Gln Ile Gly Lys His Tyr Lys Lys Val Met Tyr
355 360 365

aca cag tac gaa gat gag tcc ttc acc aaa cat aca gtg aat ccc aat 1152
Thr Gln Tyr Glu Asp Glu Ser Phe Thr Lys His Thr Val Asn Pro Asn
370 375 380

atg aaa gaa gat ggg att ttg ggt cct att atc aga gcc cag gtc aga 1200
Met Lys Glu Asp Gly Ile Leu Gly Pro Ile Ile Arg Ala Gln Val Arg
385 390 395 400

gac aca ctc aaa atc gtg ttc aaa aat atg gcc agc cgc ccc tat agc 1248
Asp Thr Leu Lys Ile Val Phe Lys Asn Met Ala Ser Arg Pro Tyr Ser
405 410 415

att tac cct cat gga gtg acc ttc tcg cct tat gaa gat gaa gtc aac 1296
Ile Tyr Pro His Gly Val Thr Phe Ser Pro Tyr Glu Asp Glu Val Asn
420 425 430

tct tct ttc acc tca ggc agg aac aac acc atg atc aga gca gtt caa 1344
Ser Ser Phe Thr Ser Gly Arg Asn Asn Thr Met Ile Arg Ala Val Gln
435 440 445

cca ggg gaa acc tat act tat aag tgg aac atc tta gag ttt gat gaa 1392
Pro Gly Glu Thr Tyr Thr Tyr Lys Trp Asn Ile Leu Glu Phe Asp Glu
450 455 460

ccc aca gaa aat gat gcc cag tgc tta aca aga cca tac tac agt gac 1440
Pro Thr Glu Asn Asp Ala Gln Cys Leu Thr Arg Pro Tyr Tyr Ser Asp
465 470 475 480

gtg gac atc atg aga gac atc gcc tct ggg cta ata gga cta ctt cta 1488

Val Asp Ile Met Arg Asp Ile Ala Ser Gly Leu Ile Gly Leu Leu Leu
                485                 490                 495

atc tgt aag agc aga tcc ctg gac agg cga gga ata cag agg gca gca      1536
Ile Cys Lys Ser Arg Ser Leu Asp Arg Arg Gly Ile Gln Arg Ala Ala
            500                 505                 510

gac atc gaa cag cag gct gtg ttt gct gtg ttt gat gag aac aaa agc      1584
Asp Ile Glu Gln Gln Ala Val Phe Ala Val Phe Asp Glu Asn Lys Ser
        515                 520                 525

tgg tac ctt gag gac aac atc aac aag ttt tgt gaa aat cct gat gag      1632
Trp Tyr Leu Glu Asp Asn Ile Asn Lys Phe Cys Glu Asn Pro Asp Glu
    530                 535                 540

gtg aaa cgt gat gac ccc aag ttt tat gaa tca aac atc atg agc act      1680
Val Lys Arg Asp Asp Pro Lys Phe Tyr Glu Ser Asn Ile Met Ser Thr
545                 550                 555                 560

atc aat ggc tat gtg cct gag agc ata act act ctt gga ttc tgc ttt      1728
Ile Asn Gly Tyr Val Pro Glu Ser Ile Thr Thr Leu Gly Phe Cys Phe
                565                 570                 575

gat gac act gtc cag tgg cac ttc tgt agt gtg ggg acc cag aat gaa      1776
Asp Asp Thr Val Gln Trp His Phe Cys Ser Val Gly Thr Gln Asn Glu
            580                 585                 590

att ttg acc atc cac ttc act ggg cac tca ttc atc tat gga aag agg      1824
Ile Leu Thr Ile His Phe Thr Gly His Ser Phe Ile Tyr Gly Lys Arg
        595                 600                 605

cat gag gac acc ttg acc ctc ttc ccc atg cgt gga gaa tct gtg acg      1872
His Glu Asp Thr Leu Thr Leu Phe Pro Met Arg Gly Glu Ser Val Thr
    610                 615                 620

gtc aca atg gat aat gtt gga act tgg atg tta act tcc atg aat tct      1920
Val Thr Met Asp Asn Val Gly Thr Trp Met Leu Thr Ser Met Asn Ser
625                 630                 635                 640

agt cca aga agc aaa aag ctg agg ctg aaa ttc agg gat gtt aaa tgt      1968
Ser Pro Arg Ser Lys Lys Leu Arg Leu Lys Phe Arg Asp Val Lys Cys
                645                 650                 655

atc cca gat gat gat gaa gac tca tat gag att ttt gaa cct cca gaa      2016
Ile Pro Asp Asp Asp Glu Asp Ser Tyr Glu Ile Phe Glu Pro Pro Glu
            660                 665                 670

tct aca gtc atg gct aca cgg aaa atg cat gat cgt tta gaa cct gaa      2064
Ser Thr Val Met Ala Thr Arg Lys Met His Asp Arg Leu Glu Pro Glu
        675                 680                 685

gat gaa gag agt gat gct gac tat gat tac cag aac aga ctg gct gca      2112
Asp Glu Glu Ser Asp Ala Asp Tyr Asp Tyr Gln Asn Arg Leu Ala Ala
    690                 695                 700

gca tta gga att agg tca ttc cga aac tca tca ttg aac cag gaa gaa      2160
Ala Leu Gly Ile Arg Ser Phe Arg Asn Ser Ser Leu Asn Gln Glu Glu
705                 710                 715                 720

gaa gag ttc aat ctt act gcc cta gct ctg gag aat ggc act gaa ttc      2208
Glu Glu Phe Asn Leu Thr Ala Leu Ala Leu Glu Asn Gly Thr Glu Phe
                725                 730                 735

gtt tct tcg aac aca gat ata att gtt ggt tca aat tat tct tcc cca      2256
Val Ser Ser Asn Thr Asp Ile Ile Val Gly Ser Asn Tyr Ser Ser Pro
            740                 745                 750

agt aat att agt aag ttc act gtc aat aac ctt gca gaa cct cag aaa      2304
Ser Asn Ile Ser Lys Phe Thr Val Asn Asn Leu Ala Glu Pro Gln Lys

755                 760                 765

gcc cct tct cac caa caa gcc acc aca gct ggt tcc cca ctg aga cac     2352
Ala Pro Ser His Gln Gln Ala Thr Thr Ala Gly Ser Pro Leu Arg His
    770                 775                 780

ctc att ggc aag aac tca gtt ctc aat tct tcc aca gca gag cat tcc     2400
Leu Ile Gly Lys Asn Ser Val Leu Asn Ser Ser Thr Ala Glu His Ser
785                 790                 795                 800

agc cca tat tct gaa gac cct ata gag gat cct cta cag cca gat gtc     2448
Ser Pro Tyr Ser Glu Asp Pro Ile Glu Asp Pro Leu Gln Pro Asp Val
                805                 810                 815

aca ggg ata cgt cta ctt tca ctt ggt gct gga gaa ttc aga agt caa     2496
Thr Gly Ile Arg Leu Leu Ser Leu Gly Ala Gly Glu Phe Arg Ser Gln
            820                 825                 830

gaa cat gct aag cgt aag gga ccc aag gta gaa aga gat caa gca gca     2544
Glu His Ala Lys Arg Lys Gly Pro Lys Val Glu Arg Asp Gln Ala Ala
        835                 840                 845

aag cac agg ttc tcc tgg atg aaa tta cta gca cat aaa gtt ggg aga     2592
Lys His Arg Phe Ser Trp Met Lys Leu Leu Ala His Lys Val Gly Arg
    850                 855                 860

cac cta agc caa gac act ggt tct cct tcc gga atg agg ccc tgg gag     2640
His Leu Ser Gln Asp Thr Gly Ser Pro Ser Gly Met Arg Pro Trp Glu
865                 870                 875                 880

gac ctt cct agc caa gac act ggt tct cct tcc aga atg agg ccc tgg     2688
Asp Leu Pro Ser Gln Asp Thr Gly Ser Pro Ser Arg Met Arg Pro Trp
                885                 890                 895

gag gac cct cct agt gat ctg tta ctc tta aaa caa agt aac tca tct     2736
Glu Asp Pro Pro Ser Asp Leu Leu Leu Leu Lys Gln Ser Asn Ser Ser
            900                 905                 910

aag att ttg gtt ggg aga tgg cat ttg gct tct gag aaa ggt agc tat     2784
Lys Ile Leu Val Gly Arg Trp His Leu Ala Ser Glu Lys Gly Ser Tyr
        915                 920                 925

gaa ata atc caa gat act gat gaa gac aca gct gtt aac aat tgg ctg     2832
Glu Ile Ile Gln Asp Thr Asp Glu Asp Thr Ala Val Asn Asn Trp Leu
    930                 935                 940

atc agc ccc cag aat gcc tca cgt gct tgg gga gaa agc acc cct ctt     2880
Ile Ser Pro Gln Asn Ala Ser Arg Ala Trp Gly Glu Ser Thr Pro Leu
945                 950                 955                 960

gcc aac aag cct gga aag cag agt ggc cac cca aag ttt cct aga gtt     2928
Ala Asn Lys Pro Gly Lys Gln Ser Gly His Pro Lys Phe Pro Arg Val
                965                 970                 975

aga cat aaa tct cta caa gta aga cag gat gga gga aag agt aga ctg     2976
Arg His Lys Ser Leu Gln Val Arg Gln Asp Gly Gly Lys Ser Arg Leu
            980                 985                 990

aag aaa agc cag ttt ctc att aag  aca cga aaa aag aaa  aaa gag aag     3024
Lys Lys Ser Gln Phe Leu Ile Lys  Thr Arg Lys Lys Lys  Lys Glu Lys
        995                 1000                1005

cac aca  cac cat gct cct tta  tct ccg agg acc ttt  cac cct cta     3069
His Thr  His His Ala Pro Leu  Ser Pro Arg Thr Phe  His Pro Leu
    1010                1015                1020

aga agt  gaa gcc tac aac aca  ttt tca gaa aga aga  ctt aag cat     3114
Arg Ser  Glu Ala Tyr Asn Thr  Phe Ser Glu Arg Arg  Leu Lys His
    1025                1030                1035

tcg ttg gtg ctt cat aaa tcc aat gaa aca tct ctt ccc aca gac 3159
Ser Leu Val Leu His Lys Ser Asn Glu Thr Ser Leu Pro Thr Asp
1040 1045 1050

ctc aat cag aca ttg ccc tct atg gat ttt ggc tgg ata gcc tca 3204
Leu Asn Gln Thr Leu Pro Ser Met Asp Phe Gly Trp Ile Ala Ser
1055 1060 1065

ctt cct gac cat aat cag aat tcc tca aat gac act ggt cag gca 3249
Leu Pro Asp His Asn Gln Asn Ser Ser Asn Asp Thr Gly Gln Ala
1070 1075 1080

agc tgt cct cca ggt ctt tat cag aca gtg ccc cca gag gaa cac 3294
Ser Cys Pro Pro Gly Leu Tyr Gln Thr Val Pro Pro Glu Glu His
1085 1090 1095

tat caa aca ttc ccc att caa gac cct gat caa atg cac tct act 3339
Tyr Gln Thr Phe Pro Ile Gln Asp Pro Asp Gln Met His Ser Thr
1100 1105 1110

tca gac ccc agt cac aga tcc tct tct cca gag ctc agt gaa atg 3384
Ser Asp Pro Ser His Arg Ser Ser Ser Pro Glu Leu Ser Glu Met
1115 1120 1125

ctt gag tat gac cga agt cac aag tcc ttc ccc aca gat ata agt 3429
Leu Glu Tyr Asp Arg Ser His Lys Ser Phe Pro Thr Asp Ile Ser
1130 1135 1140

caa atg tcc cct tcc tca gaa cat gaa gtc tgg cag aca gtc atc 3474
Gln Met Ser Pro Ser Ser Glu His Glu Val Trp Gln Thr Val Ile
1145 1150 1155

tct cca gac ctc agc cag gtg acc ctc tct cca gaa ctc agc cag 3519
Ser Pro Asp Leu Ser Gln Val Thr Leu Ser Pro Glu Leu Ser Gln
1160 1165 1170

aca aac ctc tct cca gac ctc agc cac acg act ctc tct cca gaa 3564
Thr Asn Leu Ser Pro Asp Leu Ser His Thr Thr Leu Ser Pro Glu
1175 1180 1185

ctc att cag aga aac ctt tcc cca gcc ctc ggt cag atg ccc att 3609
Leu Ile Gln Arg Asn Leu Ser Pro Ala Leu Gly Gln Met Pro Ile
1190 1195 1200

tct cca gac ctc agc cat aca acc ctt tct cca gac ctc agc cat 3654
Ser Pro Asp Leu Ser His Thr Thr Leu Ser Pro Asp Leu Ser His
1205 1210 1215

aca acc ctt tct tta gac ctc agc cag aca aac ctc tct cca gaa 3699
Thr Thr Leu Ser Leu Asp Leu Ser Gln Thr Asn Leu Ser Pro Glu
1220 1225 1230

ctc agt cag aca aac ctt tct cca gcc ctc ggt cag atg ccc ctt 3744
Leu Ser Gln Thr Asn Leu Ser Pro Ala Leu Gly Gln Met Pro Leu
1235 1240 1245

tct cca gac ctc agc cat aca acc att tct cta gac ttc agc cag 3789
Ser Pro Asp Leu Ser His Thr Thr Ile Ser Leu Asp Phe Ser Gln
1250 1255 1260

aca aac ctc tct cca gaa ctc agc cat atg act ctc tct cca gaa 3834
Thr Asn Leu Ser Pro Glu Leu Ser His Met Thr Leu Ser Pro Glu
1265 1270 1275

ctc agt cag aca aac ctt tcc cca gcc ctc ggt cag atg ccc att 3879
Leu Ser Gln Thr Asn Leu Ser Pro Ala Leu Gly Gln Met Pro Ile
1280 1285 1290

tct cca gac ctc agc cat aca acc ctt tct cta gac ttc agc cag 3924
Ser Pro Asp Leu Ser His Thr Thr Leu Ser Leu Asp Phe Ser Gln
1295 1300 1305

aca aac ctc tct cca gaa ctc agt caa aca aac ctt tcc cca gcc 3969
Thr Asn Leu Ser Pro Glu Leu Ser Gln Thr Asn Leu Ser Pro Ala
1310 1315 1320

ctc ggt cag atg ccc ctt tct cca gac ccc agc cat aca acc ctt 4014
Leu Gly Gln Met Pro Leu Ser Pro Asp Pro Ser His Thr Thr Leu
1325 1330 1335

tct cta gac ctc agc cag aca aac ctc tct cca gaa ctc agt cag 4059
Ser Leu Asp Leu Ser Gln Thr Asn Leu Ser Pro Glu Leu Ser Gln
1340 1345 1350

aca aac ctt tcc cca gac ctc agt gag atg ccc ctc ttt gca gat 4104
Thr Asn Leu Ser Pro Asp Leu Ser Glu Met Pro Leu Phe Ala Asp
1355 1360 1365

ctc agt caa att ccc ctt acc cca gac ctc gac cag atg aca ctt 4149
Leu Ser Gln Ile Pro Leu Thr Pro Asp Leu Asp Gln Met Thr Leu
1370 1375 1380

tct cca gac ctt ggt gag aca gat ctt tcc cca aac ttt ggt cag 4194
Ser Pro Asp Leu Gly Glu Thr Asp Leu Ser Pro Asn Phe Gly Gln
1385 1390 1395

atg tcc ctt tcc cca gac ctc agc cag gtg act ctc tct cca gac 4239
Met Ser Leu Ser Pro Asp Leu Ser Gln Val Thr Leu Ser Pro Asp
1400 1405 1410

atc agt gac acc acc ctt ctc ccg gat ctc agc cag ata tca cct 4284
Ile Ser Asp Thr Thr Leu Leu Pro Asp Leu Ser Gln Ile Ser Pro
1415 1420 1425

cct cca gac ctt gat cag ata ttc tac cct tct gaa tct agt cag 4329
Pro Pro Asp Leu Asp Gln Ile Phe Tyr Pro Ser Glu Ser Ser Gln
1430 1435 1440

tca ttg ctt ctt caa gaa ttt aat gag tct ttt cct tat cca gac 4374
Ser Leu Leu Leu Gln Glu Phe Asn Glu Ser Phe Pro Tyr Pro Asp
1445 1450 1455

ctt ggt cag atg cca tct cct tca tct cct act ctc aat gat act 4419
Leu Gly Gln Met Pro Ser Pro Ser Ser Pro Thr Leu Asn Asp Thr
1460 1465 1470

ttt cta tca aag gaa ttt aat cca ctg gtt ata gtg ggc ctc agt 4464
Phe Leu Ser Lys Glu Phe Asn Pro Leu Val Ile Val Gly Leu Ser
1475 1480 1485

aaa gat ggt aca gat tac att gag atc att cca aag gaa gag gtc 4509
Lys Asp Gly Thr Asp Tyr Ile Glu Ile Ile Pro Lys Glu Glu Val
1490 1495 1500

cag agc agt gaa gat gac tat gct gaa att gat tat gtg ccc tat 4554
Gln Ser Ser Glu Asp Asp Tyr Ala Glu Ile Asp Tyr Val Pro Tyr
1505 1510 1515

gat gac ccc tac aaa act gat gtt agg aca aac atc aac tcc tcc 4599
Asp Asp Pro Tyr Lys Thr Asp Val Arg Thr Asn Ile Asn Ser Ser
1520 1525 1530

aga gat cct gac aac att gca gca tgg tac ctc cgc agc aac aat 4644
Arg Asp Pro Asp Asn Ile Ala Ala Trp Tyr Leu Arg Ser Asn Asn
1535 1540 1545

gga aac aga aga aat tat tac att gct gct gaa gaa ata tcc tgg 4689

Gly Asn Arg Arg Asn Tyr Tyr Ile Ala Ala Glu Glu Ile Ser Trp
    1550                    1555                    1560

gat tat tca gaa ttt gta caa agg gaa aca gat att gaa gac tct    4734
Asp Tyr Ser Glu Phe Val Gln Arg Glu Thr Asp Ile Glu Asp Ser
    1565                    1570                    1575

gat gat att cca gaa gat acc aca tat aag aaa gta gtt ttt cga    4779
Asp Asp Ile Pro Glu Asp Thr Thr Tyr Lys Lys Val Val Phe Arg
    1580                    1585                    1590

aag tac ctc gac agc act ttt acc aaa cgt gat cct cga ggg gag    4824
Lys Tyr Leu Asp Ser Thr Phe Thr Lys Arg Asp Pro Arg Gly Glu
    1595                    1600                    1605

tat gaa gag cat ctc gga att ctt ggt cct att atc aga gct gaa    4869
Tyr Glu Glu His Leu Gly Ile Leu Gly Pro Ile Ile Arg Ala Glu
    1610                    1615                    1620

gtg gat gat gtt atc caa gtt cgt ttt aaa aat tta gca tcc aga    4914
Val Asp Asp Val Ile Gln Val Arg Phe Lys Asn Leu Ala Ser Arg
    1625                    1630                    1635

ccg tat tct cta cat gcc cat gga ctt tcc tat gaa aaa tca tca    4959
Pro Tyr Ser Leu His Ala His Gly Leu Ser Tyr Glu Lys Ser Ser
    1640                    1645                    1650

gag gga aag act tat gaa gat gac tct cct gaa tgg ttt aag gaa    5004
Glu Gly Lys Thr Tyr Glu Asp Asp Ser Pro Glu Trp Phe Lys Glu
    1655                    1660                    1665

gat aat gct gtt cag cca aat agc agt tat acc tac gta tgg cat    5049
Asp Asn Ala Val Gln Pro Asn Ser Ser Tyr Thr Tyr Val Trp His
    1670                    1675                    1680

gcc act gag cga tca ggg cca gaa agt cct ggc tct gcc tgt cgg    5094
Ala Thr Glu Arg Ser Gly Pro Glu Ser Pro Gly Ser Ala Cys Arg
    1685                    1690                    1695

gct tgg gcc tac tac tca gct gtg aac cca gaa aaa gat att cac    5139
Ala Trp Ala Tyr Tyr Ser Ala Val Asn Pro Glu Lys Asp Ile His
    1700                    1705                    1710

tca ggc ttg ata ggt ccc ctc cta atc tgc caa aaa gga ata cta    5184
Ser Gly Leu Ile Gly Pro Leu Leu Ile Cys Gln Lys Gly Ile Leu
    1715                    1720                    1725

cat aag gac agc aac atg cct gtg gac atg aga gaa ttt gtc tta    5229
His Lys Asp Ser Asn Met Pro Val Asp Met Arg Glu Phe Val Leu
    1730                    1735                    1740

cta ttt atg acc ttt gat gaa aag aag agc tgg tac tat gaa aag    5274
Leu Phe Met Thr Phe Asp Glu Lys Lys Ser Trp Tyr Tyr Glu Lys
    1745                    1750                    1755

aag tcc cga agt tct tgg aga ctc aca tcc tca gaa atg aaa aaa    5319
Lys Ser Arg Ser Ser Trp Arg Leu Thr Ser Ser Glu Met Lys Lys
    1760                    1765                    1770

tcc cat gag ttt cac gcc att aat ggg atg atc tac agc ttg cct    5364
Ser His Glu Phe His Ala Ile Asn Gly Met Ile Tyr Ser Leu Pro
    1775                    1780                    1785

ggc ctg aaa atg tat gag caa gag tgg gtg agg tta cac ctg ctg    5409
Gly Leu Lys Met Tyr Glu Gln Glu Trp Val Arg Leu His Leu Leu
    1790                    1795                    1800

aac ata ggc ggc tcc caa gac att cac gtg gtt cac ttt cac ggc    5454
Asn Ile Gly Gly Ser Gln Asp Ile His Val Val His Phe His Gly

1805                    1810                    1815

cag acc ttg ctg gaa aat ggc aat aaa cag cac cag tta ggg gtc    5499
Gln Thr Leu Leu Glu Asn Gly Asn Lys Gln His Gln Leu Gly Val
1820                    1825                    1830

tgg ccc ctt ctg cct ggt tca ttt aaa act ctt gaa atg aag gca    5544
Trp Pro Leu Leu Pro Gly Ser Phe Lys Thr Leu Glu Met Lys Ala
1835                    1840                    1845

tca aaa cct ggc tgg tgg ctc cta aac aca gag gtt gga gaa aac    5589
Ser Lys Pro Gly Trp Trp Leu Leu Asn Thr Glu Val Gly Glu Asn
1850                    1855                    1860

cag aga gca ggg atg caa acg cca ttt ctt atc atg gac aga gac    5634
Gln Arg Ala Gly Met Gln Thr Pro Phe Leu Ile Met Asp Arg Asp
1865                    1870                    1875

tgt agg atg cca atg gga cta agc act ggt atc ata tct gat tca    5679
Cys Arg Met Pro Met Gly Leu Ser Thr Gly Ile Ile Ser Asp Ser
1880                    1885                    1890

cag atc aag gct tca gag ttt ctg ggt tac tgg gag ccc aga tta    5724
Gln Ile Lys Ala Ser Glu Phe Leu Gly Tyr Trp Glu Pro Arg Leu
1895                    1900                    1905

gca aga tta aac aat ggt gga tct tat aat gct tgg agt gta gaa    5769
Ala Arg Leu Asn Asn Gly Gly Ser Tyr Asn Ala Trp Ser Val Glu
1910                    1915                    1920

aaa ctt gca gca gaa ttt gcc tct aaa cct tgg atc cag gtg gac    5814
Lys Leu Ala Ala Glu Phe Ala Ser Lys Pro Trp Ile Gln Val Asp
1925                    1930                    1935

atg caa aag gaa gtc ata atc aca ggg atc cag acc caa ggt gcc    5859
Met Gln Lys Glu Val Ile Ile Thr Gly Ile Gln Thr Gln Gly Ala
1940                    1945                    1950

aaa cac tac ctg aag tcc tgc tat acc aca gag ttc tat gta gct    5904
Lys His Tyr Leu Lys Ser Cys Tyr Thr Thr Glu Phe Tyr Val Ala
1955                    1960                    1965

tac agt tcc aac cag atc aac tgg cag atc ttc aaa ggg aac agc    5949
Tyr Ser Ser Asn Gln Ile Asn Trp Gln Ile Phe Lys Gly Asn Ser
1970                    1975                    1980

aca agg aat gtg atg tat ttt aat ggc aat tca gat gcc tct aca    5994
Thr Arg Asn Val Met Tyr Phe Asn Gly Asn Ser Asp Ala Ser Thr
1985                    1990                    1995

ata aaa gag aat cag ttt gac cca cct att gtg gct aga tat att    6039
Ile Lys Glu Asn Gln Phe Asp Pro Pro Ile Val Ala Arg Tyr Ile
2000                    2005                    2010

agg atc tct cca act cga gcc tat aac aga cct acc ctt cga ttg    6084
Arg Ile Ser Pro Thr Arg Ala Tyr Asn Arg Pro Thr Leu Arg Leu
2015                    2020                    2025

gaa ctg caa ggt tgt gag gta aat gga tgt tcc aca ccc ctg ggt    6129
Glu Leu Gln Gly Cys Glu Val Asn Gly Cys Ser Thr Pro Leu Gly
2030                    2035                    2040

atg gaa aat gga aag ata gaa aac aag caa atc aca gct tct tcg    6174
Met Glu Asn Gly Lys Ile Glu Asn Lys Gln Ile Thr Ala Ser Ser
2045                    2050                    2055

ttt aag aaa tct tgg tgg gga gat tac tgg gaa ccc ttc cgt gcc    6219
Phe Lys Lys Ser Trp Trp Gly Asp Tyr Trp Glu Pro Phe Arg Ala
2060                    2065                    2070

Gly Ala Ser Tyr Leu Asp His Thr Phe Pro Ala Glu Lys Met Asp Asp
            100                 105                 110

Ala Val Ala Pro Gly Arg Glu Tyr Thr Tyr Glu Trp Ser Ile Ser Glu
        115                 120                 125

Asp Ser Gly Pro Thr His Asp Asp Pro Pro Cys Leu Thr His Ile Tyr
    130                 135                 140

Tyr Ser His Glu Asn Leu Ile Glu Asp Phe Asn Ser Gly Leu Ile Gly
145                 150                 155                 160

Pro Leu Leu Ile Cys Lys Lys Gly Thr Leu Thr Glu Gly Gly Thr Gln
                165                 170                 175

Lys Thr Phe Asp Lys Gln Ile Val Leu Leu Phe Ala Val Phe Asp Glu
            180                 185                 190

Ser Lys Ser Trp Ser Gln Ser Ser Ser Leu Met Tyr Thr Val Asn Gly
        195                 200                 205

Tyr Val Asn Gly Thr Met Pro Asp Ile Thr Val Cys Ala His Asp His
    210                 215                 220

Ile Ser Trp His Leu Leu Gly Met Ser Ser Gly Pro Glu Leu Phe Ser
225                 230                 235                 240

Ile His Phe Asn Gly Gln Val Leu Glu Gln Asn His His Lys Val Ser
                245                 250                 255

Ala Ile Thr Leu Val Ser Ala Thr Ser Thr Thr Ala Asn Met Thr Val
            260                 265                 270

Gly Pro Glu Gly Lys Trp Ile Ile Ser Ser Leu Thr Pro Lys His Leu
        275                 280                 285

Gln Ala Gly Met Gln Ala Tyr Ile Asp Ile Lys Asn Cys Pro Lys Lys
    290                 295                 300

Thr Arg Asn Leu Lys Lys Ile Thr Arg Glu Gln Arg Arg His Met Lys
305                 310                 315                 320

Arg Trp Glu Tyr Phe Ile Ala Ala Glu Glu Val Ile Trp Asp Tyr Ala
                325                 330                 335

Pro Val Ile Pro Ala Asn Met Asp Lys Lys Tyr Arg Ser Gln His Leu
            340                 345                 350

Asp Asn Phe Ser Asn Gln Ile Gly Lys His Tyr Lys Lys Val Met Tyr
        355                 360                 365

Thr Gln Tyr Glu Asp Glu Ser Phe Thr Lys His Thr Val Asn Pro Asn

370                 375                 380

Met Lys Glu Asp Gly Ile Leu Gly Pro Ile Ile Arg Ala Gln Val Arg
385                 390                 395                 400

Asp Thr Leu Lys Ile Val Phe Lys Asn Met Ala Ser Arg Pro Tyr Ser
                405                 410                 415

Ile Tyr Pro His Gly Val Thr Phe Ser Pro Tyr Glu Asp Glu Val Asn
            420                 425                 430

Ser Ser Phe Thr Ser Gly Arg Asn Asn Thr Met Ile Arg Ala Val Gln
        435                 440                 445

Pro Gly Glu Thr Tyr Thr Tyr Lys Trp Asn Ile Leu Glu Phe Asp Glu
    450                 455                 460

Pro Thr Glu Asn Asp Ala Gln Cys Leu Thr Arg Pro Tyr Tyr Ser Asp
465                 470                 475                 480

Val Asp Ile Met Arg Asp Ile Ala Ser Gly Leu Ile Gly Leu Leu Leu
                485                 490                 495

Ile Cys Lys Ser Arg Ser Leu Asp Arg Arg Gly Ile Gln Arg Ala Ala
            500                 505                 510

Asp Ile Glu Gln Gln Ala Val Phe Ala Val Phe Asp Glu Asn Lys Ser
        515                 520                 525

Trp Tyr Leu Glu Asp Asn Ile Asn Lys Phe Cys Glu Asn Pro Asp Glu
    530                 535                 540

Val Lys Arg Asp Asp Pro Lys Phe Tyr Glu Ser Asn Ile Met Ser Thr
545                 550                 555                 560

Ile Asn Gly Tyr Val Pro Glu Ser Ile Thr Thr Leu Gly Phe Cys Phe
                565                 570                 575

Asp Asp Thr Val Gln Trp His Phe Cys Ser Val Gly Thr Gln Asn Glu
            580                 585                 590

Ile Leu Thr Ile His Phe Thr Gly His Ser Phe Ile Tyr Gly Lys Arg
        595                 600                 605

His Glu Asp Thr Leu Thr Leu Phe Pro Met Arg Gly Glu Ser Val Thr
    610                 615                 620

Val Thr Met Asp Asn Val Gly Thr Trp Met Leu Thr Ser Met Asn Ser
625                 630                 635                 640

Ser Pro Arg Ser Lys Lys Leu Arg Leu Lys Phe Arg Asp Val Lys Cys
                645                 650                 655

Ile Pro Asp Asp Asp Glu Asp Ser Tyr Glu Ile Phe Glu Pro Pro Glu
            660                 665                 670

Ser Thr Val Met Ala Thr Arg Lys Met His Asp Arg Leu Glu Pro Glu
        675                 680                 685

Asp Glu Glu Ser Asp Ala Asp Tyr Asp Tyr Gln Asn Arg Leu Ala Ala
    690                 695                 700

Ala Leu Gly Ile Arg Ser Phe Arg Asn Ser Ser Leu Asn Gln Glu Glu
705                 710                 715                 720

Glu Glu Phe Asn Leu Thr Ala Leu Ala Leu Glu Asn Gly Thr Glu Phe
                725                 730                 735

Val Ser Ser Asn Thr Asp Ile Ile Val Gly Ser Asn Tyr Ser Ser Pro
            740                 745                 750

Ser Asn Ile Ser Lys Phe Thr Val Asn Asn Leu Ala Glu Pro Gln Lys
        755                 760                 765

Ala Pro Ser His Gln Gln Ala Thr Thr Ala Gly Ser Pro Leu Arg His
    770                 775                 780

Leu Ile Gly Lys Asn Ser Val Leu Asn Ser Ser Thr Ala Glu His Ser
785                 790                 795                 800

Ser Pro Tyr Ser Glu Asp Pro Ile Glu Asp Pro Leu Gln Pro Asp Val
                805                 810                 815

Thr Gly Ile Arg Leu Leu Ser Leu Gly Ala Gly Glu Phe Arg Ser Gln
            820                 825                 830

Glu His Ala Lys Arg Lys Gly Pro Lys Val Glu Arg Asp Gln Ala Ala
        835                 840                 845

Lys His Arg Phe Ser Trp Met Lys Leu Leu Ala His Lys Val Gly Arg
    850                 855                 860

His Leu Ser Gln Asp Thr Gly Ser Pro Ser Gly Met Arg Pro Trp Glu
865                 870                 875                 880

Asp Leu Pro Ser Gln Asp Thr Gly Ser Pro Ser Arg Met Arg Pro Trp
                885                 890                 895

Glu Asp Pro Pro Ser Asp Leu Leu Leu Leu Lys Gln Ser Asn Ser Ser
            900                 905                 910

Lys Ile Leu Val Gly Arg Trp His Leu Ala Ser Glu Lys Gly Ser Tyr
        915                 920                 925

Glu Ile Ile Gln Asp Thr Asp Glu Asp Thr Ala Val Asn Asn Trp Leu
930 935 940

Ile Ser Pro Gln Asn Ala Ser Arg Ala Trp Gly Glu Ser Thr Pro Leu
945 950 955 960

Ala Asn Lys Pro Gly Lys Gln Ser Gly His Pro Lys Phe Pro Arg Val
965 970 975

Arg His Lys Ser Leu Gln Val Arg Gln Asp Gly Gly Lys Ser Arg Leu
980 985 990

Lys Lys Ser Gln Phe Leu Ile Lys Thr Arg Lys Lys Lys Lys Glu Lys
995 1000 1005

His Thr His His Ala Pro Leu Ser Pro Arg Thr Phe His Pro Leu
1010 1015 1020

Arg Ser Glu Ala Tyr Asn Thr Phe Ser Glu Arg Arg Leu Lys His
1025 1030 1035

Ser Leu Val Leu His Lys Ser Asn Glu Thr Ser Leu Pro Thr Asp
1040 1045 1050

Leu Asn Gln Thr Leu Pro Ser Met Asp Phe Gly Trp Ile Ala Ser
1055 1060 1065

Leu Pro Asp His Asn Gln Asn Ser Ser Asn Asp Thr Gly Gln Ala
1070 1075 1080

Ser Cys Pro Pro Gly Leu Tyr Gln Thr Val Pro Pro Glu Glu His
1085 1090 1095

Tyr Gln Thr Phe Pro Ile Gln Asp Pro Asp Gln Met His Ser Thr
1100 1105 1110

Ser Asp Pro Ser His Arg Ser Ser Ser Pro Glu Leu Ser Glu Met
1115 1120 1125

Leu Glu Tyr Asp Arg Ser His Lys Ser Phe Pro Thr Asp Ile Ser
1130 1135 1140

Gln Met Ser Pro Ser Ser Glu His Glu Val Trp Gln Thr Val Ile
1145 1150 1155

Ser Pro Asp Leu Ser Gln Val Thr Leu Ser Pro Glu Leu Ser Gln
1160 1165 1170

Thr Asn Leu Ser Pro Asp Leu Ser His Thr Thr Leu Ser Pro Glu
1175 1180 1185

Leu Ile Gln Arg Asn Leu Ser Pro Ala Leu Gly Gln Met Pro Ile
1190 1195 1200

Ser Pro Asp Leu Ser His Thr Thr Leu Ser Pro Asp Leu Ser His
1205 1210 1215

Thr Thr Leu Ser Leu Asp Leu Ser Gln Thr Asn Leu Ser Pro Glu
1220 1225 1230

Leu Ser Gln Thr Asn Leu Ser Pro Ala Leu Gly Gln Met Pro Leu
1235 1240 1245

Ser Pro Asp Leu Ser His Thr Thr Ile Ser Leu Asp Phe Ser Gln
1250 1255 1260

Thr Asn Leu Ser Pro Glu Leu Ser His Met Thr Leu Ser Pro Glu
1265 1270 1275

Leu Ser Gln Thr Asn Leu Ser Pro Ala Leu Gly Gln Met Pro Ile
1280 1285 1290

Ser Pro Asp Leu Ser His Thr Thr Leu Ser Leu Asp Phe Ser Gln
1295 1300 1305

Thr Asn Leu Ser Pro Glu Leu Ser Gln Thr Asn Leu Ser Pro Ala
1310 1315 1320

Leu Gly Gln Met Pro Leu Ser Pro Asp Pro Ser His Thr Thr Leu
1325 1330 1335

Ser Leu Asp Leu Ser Gln Thr Asn Leu Ser Pro Glu Leu Ser Gln
1340 1345 1350

Thr Asn Leu Ser Pro Asp Leu Ser Glu Met Pro Leu Phe Ala Asp
1355 1360 1365

Leu Ser Gln Ile Pro Leu Thr Pro Asp Leu Asp Gln Met Thr Leu
1370 1375 1380

Ser Pro Asp Leu Gly Glu Thr Asp Leu Ser Pro Asn Phe Gly Gln
1385 1390 1395

Met Ser Leu Ser Pro Asp Leu Ser Gln Val Thr Leu Ser Pro Asp
1400 1405 1410

Ile Ser Asp Thr Thr Leu Leu Pro Asp Leu Ser Gln Ile Ser Pro
1415 1420 1425

Pro Pro Asp Leu Asp Gln Ile Phe Tyr Pro Ser Glu Ser Ser Gln
1430 1435 1440

Ser Leu Leu Leu Gln Glu Phe Asn Glu Ser Phe Pro Tyr Pro Asp

1445                    1450                    1455

Leu Gly  Gln Met Pro Ser Pro  Ser Ser Pro Thr Leu  Asn Asp Thr
    1460                    1465                    1470

Phe Leu  Ser Lys Glu Phe Asn  Pro Leu Val Ile Val  Gly Leu Ser
    1475                    1480                    1485

Lys Asp  Gly Thr Asp Tyr Ile  Glu Ile Ile Pro Lys  Glu Glu Val
    1490                    1495                    1500

Gln Ser  Ser Glu Asp Asp Tyr  Ala Glu Ile Asp Tyr  Val Pro Tyr
    1505                    1510                    1515

Asp Asp  Pro Tyr Lys Thr Asp  Val Arg Thr Asn Ile  Asn Ser Ser
    1520                    1525                    1530

Arg Asp  Pro Asp Asn Ile Ala  Ala Trp Tyr Leu Arg  Ser Asn Asn
    1535                    1540                    1545

Gly Asn  Arg Arg Asn Tyr Tyr  Ile Ala Ala Glu Glu  Ile Ser Trp
    1550                    1555                    1560

Asp Tyr  Ser Glu Phe Val Gln  Arg Glu Thr Asp Ile  Glu Asp Ser
    1565                    1570                    1575

Asp Asp  Ile Pro Glu Asp Thr  Thr Tyr Lys Lys Val  Val Phe Arg
    1580                    1585                    1590

Lys Tyr  Leu Asp Ser Thr Phe  Thr Lys Arg Asp Pro  Arg Gly Glu
    1595                    1600                    1605

Tyr Glu  Glu His Leu Gly Ile  Leu Gly Pro Ile Ile  Arg Ala Glu
    1610                    1615                    1620

Val Asp  Asp Val Ile Gln Val  Arg Phe Lys Asn Leu  Ala Ser Arg
    1625                    1630                    1635

Pro Tyr  Ser Leu His Ala His  Gly Leu Ser Tyr Glu  Lys Ser Ser
    1640                    1645                    1650

Glu Gly  Lys Thr Tyr Glu Asp  Asp Ser Pro Glu Trp  Phe Lys Glu
    1655                    1660                    1665

Asp Asn  Ala Val Gln Pro Asn  Ser Ser Tyr Thr Tyr  Val Trp His
    1670                    1675                    1680

Ala Thr  Glu Arg Ser Gly Pro  Glu Ser Pro Gly Ser  Ala Cys Arg
    1685                    1690                    1695

Ala Trp  Ala Tyr Tyr Ser Ala  Val Asn Pro Glu Lys  Asp Ile His
    1700                    1705                    1710

Ser Gly Leu Ile Gly Pro Leu Leu Ile Cys Gln Lys Gly Ile Leu
    1715                1720                1725

His Lys Asp Ser Asn Met Pro Val Asp Met Arg Glu Phe Val Leu
    1730                1735                1740

Leu Phe Met Thr Phe Asp Glu Lys Lys Ser Trp Tyr Tyr Glu Lys
    1745                1750                1755

Lys Ser Arg Ser Ser Trp Arg Leu Thr Ser Ser Glu Met Lys Lys
    1760                1765                1770

Ser His Glu Phe His Ala Ile Asn Gly Met Ile Tyr Ser Leu Pro
    1775                1780                1785

Gly Leu Lys Met Tyr Glu Gln Glu Trp Val Arg Leu His Leu Leu
    1790                1795                1800

Asn Ile Gly Gly Ser Gln Asp Ile His Val Val His Phe His Gly
    1805                1810                1815

Gln Thr Leu Leu Glu Asn Gly Asn Lys Gln His Gln Leu Gly Val
    1820                1825                1830

Trp Pro Leu Leu Pro Gly Ser Phe Lys Thr Leu Glu Met Lys Ala
    1835                1840                1845

Ser Lys Pro Gly Trp Trp Leu Leu Asn Thr Glu Val Gly Glu Asn
    1850                1855                1860

Gln Arg Ala Gly Met Gln Thr Pro Phe Leu Ile Met Asp Arg Asp
    1865                1870                1875

Cys Arg Met Pro Met Gly Leu Ser Thr Gly Ile Ile Ser Asp Ser
    1880                1885                1890

Gln Ile Lys Ala Ser Glu Phe Leu Gly Tyr Trp Glu Pro Arg Leu
    1895                1900                1905

Ala Arg Leu Asn Asn Gly Gly Ser Tyr Asn Ala Trp Ser Val Glu
    1910                1915                1920

Lys Leu Ala Ala Glu Phe Ala Ser Lys Pro Trp Ile Gln Val Asp
    1925                1930                1935

Met Gln Lys Glu Val Ile Ile Thr Gly Ile Gln Thr Gln Gly Ala
    1940                1945                1950

Lys His Tyr Leu Lys Ser Cys Tyr Thr Thr Glu Phe Tyr Val Ala
    1955                1960                1965

Tyr Ser Ser Asn Gln Ile Asn Trp Gln Ile Phe Lys Gly Asn Ser
1970 1975 1980

Thr Arg Asn Val Met Tyr Phe Asn Gly Asn Ser Asp Ala Ser Thr
1985 1990 1995

Ile Lys Glu Asn Gln Phe Asp Pro Pro Ile Val Ala Arg Tyr Ile
2000 2005 2010

Arg Ile Ser Pro Thr Arg Ala Tyr Asn Arg Pro Thr Leu Arg Leu
2015 2020 2025

Glu Leu Gln Gly Cys Glu Val Asn Gly Cys Ser Thr Pro Leu Gly
2030 2035 2040

Met Glu Asn Gly Lys Ile Glu Asn Lys Gln Ile Thr Ala Ser Ser
2045 2050 2055

Phe Lys Lys Ser Trp Trp Gly Asp Tyr Trp Glu Pro Phe Arg Ala
2060 2065 2070

Arg Leu Asn Ala Gln Gly Arg Val Asn Ala Trp Gln Ala Lys Ala
2075 2080 2085

Asn Asn Asn Lys Gln Trp Leu Glu Ile Asp Leu Leu Lys Ile Lys
2090 2095 2100

Lys Ile Thr Ala Ile Ile Thr Gln Gly Cys Lys Ser Leu Ser Ser
2105 2110 2115

Glu Met Tyr Val Lys Ser Tyr Thr Ile His Tyr Ser Glu Gln Gly
2120 2125 2130

Val Glu Trp Lys Pro Tyr Arg Leu Lys Ser Ser Met Val Asp Lys
2135 2140 2145

Ile Phe Glu Gly Asn Thr Asn Thr Lys Gly His Val Lys Asn Phe
2150 2155 2160

Phe Asn Pro Pro Ile Ile Ser Arg Phe Ile Arg Val Ile Pro Lys
2165 2170 2175

Thr Trp Asn Gln Ser Ile Ala Leu Arg Leu Glu Leu Phe Gly Cys
2180 2185 2190

Asp Ile Tyr
2195

<210> 5
<211> 1046
<212> PRT
<213> homo sapiens

<400> 5

Lys Glu Lys His Tyr Tyr Ile Gly Ile Ile Glu Thr Thr Trp Asp Tyr
1               5                   10                  15

Ala Ser Asp His Gly Glu Lys Lys Leu Ile Ser Val Asp Thr Glu His
            20                  25                  30

Ser Asn Ile Tyr Leu Gln Asn Gly Pro Asp Arg Ile Gly Arg Leu Tyr
        35                  40                  45

Lys Lys Ala Leu Tyr Leu Gln Tyr Thr Asp Glu Thr Phe Arg Thr Thr
    50                  55                  60

Ile Glu Lys Pro Val Trp Leu Gly Phe Leu Gly Pro Ile Ile Lys Ala
65                  70                  75                  80

Glu Thr Gly Asp Lys Val Tyr Val His Leu Lys Asn Leu Ala Ser Arg
                85                  90                  95

Pro Tyr Thr Phe His Ser His Gly Ile Thr Tyr Tyr Lys Glu His Glu
            100                 105                 110

Gly Ala Ile Tyr Pro Asp Asn Thr Thr Asp Phe Gln Arg Ala Asp Asp
        115                 120                 125

Lys Val Tyr Pro Gly Glu Gln Tyr Thr Tyr Met Leu Leu Ala Thr Glu
    130                 135                 140

Glu Gln Ser Pro Gly Glu Gly Asp Gly Asn Cys Val Thr Arg Ile Tyr
145                 150                 155                 160

His Ser His Ile Asp Ala Pro Lys Asp Ile Ala Ser Gly Leu Ile Gly
                165                 170                 175

Pro Leu Ile Ile Cys Lys Lys Asp Ser Leu Asp Lys Glu Lys Glu Lys
            180                 185                 190

His Ile Asp Arg Glu Phe Val Val Met Phe Ser Val Val Asp Glu Asn
        195                 200                 205

Phe Ser Trp Tyr Leu Glu Asp Asn Ile Lys Thr Tyr Cys Ser Glu Pro
    210                 215                 220

Glu Lys Val Asp Lys Asp Asn Glu Asp Phe Gln Glu Ser Asn Arg Met
225                 230                 235                 240

Tyr Ser Val Asn Gly Tyr Thr Phe Gly Ser Leu Pro Gly Leu Ser Met
                245                 250                 255

Cys Ala Glu Asp Arg Val Lys Trp Tyr Leu Phe Gly Met Gly Asn Glu
            260                 265                 270

Val Asp Val His Ala Ala Phe Phe His Gly Gln Ala Leu Thr Asn Lys
        275                 280                 285

Asn Tyr Arg Ile Asp Thr Ile Asn Leu Phe Pro Ala Thr Leu Phe Asp
    290                 295                 300

Ala Tyr Met Val Ala Gln Asn Pro Gly Glu Trp Met Leu Ser Cys Gln
305                 310                 315                 320

Asn Leu Asn His Leu Lys Ala Gly Leu Gln Ala Phe Phe Gln Val Gln
                325                 330                 335

Glu Cys Asn Lys Ser Ser Ser Lys Asp Asn Ile Arg Gly Lys His Val
            340                 345                 350

Arg His Tyr Tyr Ile Ala Ala Glu Glu Ile Ile Trp Asn Tyr Ala Pro
        355                 360                 365

Ser Gly Ile Asp Ile Phe Thr Lys Glu Asn Leu Thr Ala Pro Gly Ser
    370                 375                 380

Asp Ser Ala Val Phe Phe Glu Gln Gly Thr Thr Arg Ile Gly Gly Ser
385                 390                 395                 400

Tyr Lys Lys Leu Val Tyr Arg Glu Tyr Thr Asp Ala Ser Phe Thr Asn
                405                 410                 415

Arg Lys Glu Arg Gly Pro Glu Glu Glu His Leu Gly Ile Leu Gly Pro
            420                 425                 430

Val Ile Trp Ala Glu Val Gly Asp Thr Ile Arg Val Thr Phe His Asn
        435                 440                 445

Lys Gly Ala Tyr Pro Leu Ser Ile Glu Pro Ile Gly Val Arg Phe Asn
    450                 455                 460

Lys Asn Asn Glu Gly Thr Tyr Tyr Ser Pro Asn Tyr Asn Pro Gln Ser
465                 470                 475                 480

Arg Ser Val Pro Pro Ser Ala Ser His Val Ala Pro Thr Glu Thr Phe
                485                 490                 495

Thr Tyr Glu Trp Thr Val Pro Lys Glu Val Gly Pro Thr Asn Ala Asp
            500                 505                 510

Pro Val Cys Leu Ala Lys Met Tyr Tyr Ser Ala Val Asp Pro Thr Lys
        515                 520                 525

Asp Ile Phe Thr Gly Leu Ile Gly Pro Met Lys Ile Cys Lys Lys Gly
    530                 535                 540

Ser Leu His Ala Asn Gly Arg Gln Lys Asp Val Asp Lys Glu Phe Tyr
545                 550                 555                 560

Leu Phe Pro Thr Val Phe Asp Glu Asn Glu Ser Leu Leu Leu Glu Asp
                565                 570                 575

Asn Ile Arg Met Phe Thr Thr Ala Pro Asp Gln Val Asp Lys Glu Asp
            580                 585                 590

Glu Asp Phe Gln Glu Ser Asn Lys Met His Ser Met Asn Gly Phe Met
        595                 600                 605

Tyr Gly Asn Gln Pro Gly Leu Thr Met Cys Lys Gly Asp Ser Val Val
    610                 615                 620

Trp Tyr Leu Phe Ser Ala Gly Asn Glu Ala Asp Val His Gly Ile Tyr
625                 630                 635                 640

Phe Ser Gly Asn Thr Tyr Leu Trp Arg Gly Glu Arg Arg Asp Thr Ala
                645                 650                 655

Asn Leu Phe Pro Gln Thr Ser Leu Thr Leu His Met Trp Pro Asp Thr
            660                 665                 670

Glu Gly Thr Phe Asn Val Glu Cys Leu Thr Thr Asp His Tyr Thr Gly
        675                 680                 685

Gly Met Lys Gln Lys Tyr Thr Val Asn Gln Cys Arg Arg Gln Ser Glu
    690                 695                 700

Asp Ser Thr Phe Tyr Leu Gly Glu Arg Thr Tyr Tyr Ile Ala Ala Val
705                 710                 715                 720

Glu Val Glu Trp Asp Tyr Ser Pro Gln Arg Glu Trp Glu Lys Glu Leu
                725                 730                 735

His His Leu Gln Glu Gln Asn Val Ser Asn Ala Phe Leu Asp Lys Gly
            740                 745                 750

Glu Phe Tyr Ile Gly Ser Lys Tyr Lys Lys Val Val Tyr Arg Gln Tyr
        755                 760                 765

Thr Asp Ser Thr Phe Arg Val Pro Val Glu Arg Lys Ala Glu Glu Glu
    770                 775                 780

His Leu Gly Ile Leu Gly Pro Gln Leu His Ala Asp Val Gly Asp Lys
785                 790                 795                 800

Val Lys Ile Ile Phe Lys Asn Met Ala Thr Arg Pro Tyr Ser Ile His
                805                 810                 815

Ala His Gly Val Gln Thr Glu Ser Ser Thr Val Thr Pro Thr Leu Pro
            820                 825                 830

Gly Glu Thr Leu Thr Tyr Val Trp Lys Ile Pro Glu Arg Ser Gly Ala
        835                 840                 845

Gly Thr Glu Asp Ser Ala Cys Ile Pro Trp Ala Tyr Tyr Ser Thr Val
    850                 855                 860

Asp Gln Val Lys Asp Leu Tyr Ser Gly Leu Ile Gly Pro Leu Ile Val
865                 870                 875                 880

Cys Arg Arg Pro Tyr Leu Lys Val Phe Asn Pro Arg Arg Lys Leu Glu
                885                 890                 895

Phe Ala Leu Leu Phe Leu Val Phe Asp Glu Asn Glu Ser Trp Tyr Leu
            900                 905                 910

Asp Asp Asn Ile Lys Thr Tyr Ser Asp His Pro Glu Lys Val Asn Lys
        915                 920                 925

Asp Asp Glu Glu Phe Ile Glu Ser Asn Lys Met His Ala Ile Asn Gly
    930                 935                 940

Arg Met Phe Gly Asn Leu Gln Gly Leu Thr Met His Val Gly Asp Glu
945                 950                 955                 960

Val Asn Trp Tyr Leu Met Gly Met Gly Asn Glu Ile Asp Leu His Thr
                965                 970                 975

Val His Phe His Gly His Ser Phe Gln Tyr Lys His Arg Gly Val Tyr
            980                 985                 990

Ser Ser Asp Val Phe Asp Ile Phe  Pro Gly Thr Tyr Gln  Thr Leu Glu
        995                 1000                 1005

Met Phe  Pro Arg Thr Pro Gly  Ile Trp Leu Leu His  Cys His Val
    1010                 1015                 1020

Thr Asp  His Ile His Ala Gly  Met Glu Thr Thr Tyr  Thr Val Leu
    1025                 1030                 1035

Gln Asn  Glu Asp Thr Lys Ser  Gly
    1040                 1045

<210>  6
<211>  38
<212>  DNA
<213>  artificial sequence

<220>
<223>  L649H forward primer

<400>  6

## Claims

1. A method of producing a modified homologue of human ceruloplasmin, comprising adding at least one metal binding site to a homologue of human ceruloplasmin, wherein said metal binding site corresponds to a metal binding site or to an incomplete metal binding site in human ceruloplasmin.

2. A method according to claim 1, comprising the following steps:
(a) identifying the amino acids in the amino acid sequence of the homologue of human ceruloplasmin, which correspond to the amino acids in the amino acid sequence of human ceruloplasmin forming a metal binding site or an incomplete metal binding site in human ceruloplasmin, wherein the identified amino acids in the amino acid sequence of the homologue of human ceruloplasmin do not form a metal binding site;
(b) identifying one or more mutations in the amino acids identified in step (a), which would add a metal binding site to the homologue of human ceruloplasmin;
(c) preparing a polynucleotide encoding a modified homologue of human ceruloplasmin, wherein the modified homologue comprises at least one metal binding site according to (b); and
(d) expressing the polynucleotide in a host cell and optionally recovering the modified homologue.

3. A method according to claim 1 or 2, wherein the metal binding site or the incomplete metal binding site in human ceruloplasmin is formed by one of the following groups of amino acids:
(i) His637, Cys680, His 685 and Met 690;
(ii) Glu597, His602, Asp684 and Glu971;
(iii) Glu935, His940, Asp1025 and Glu272; or
(iv) Glu236, Tyr241, Asn323 and Glu633;
wherein the amino acid numbering refers to the amino acid sequence as shown in SEQ ID NO:5.

4. A method according to any one of claims 1 to 3, wherein the homologue of human ceruloplasmin is human blood coagulation Factor V or human blood coagulation Factor VIII.

5. A method according to any one of claims 1 to 4, wherein the homologue of human ceruloplasmin is human blood coagulation Factor VIII, and one or more of the following groups (i) to (iv) of substitutions in the amino acid sequence of human blood coagulation Factor VIII are effected:
(i) Leu649→His and Phe697→His;
(ii) Ala610→Glu and Asn1950→Glu;
(iii) optionally Glu2004→Asp and Thr263→Glu; and/or (iv) Ala227→Glu, Ser314→Asp and Gln645→Glu;
wherein the amino acid numbering refers to the amino acid sequence as shown in SEQ ID NO:2.

6. A method according to any one of claims 1 to 4, wherein the homologue of human ceruloplasmin is human blood coagulation Factor V, and one or more of the following groups (i) to (iv) of substitutions in the amino acid sequence of human blood coagulation Factor V are effected:
(i) Leu594→His, Ser637→Cys, Pro642→His and Leu647→Met;
(ii) Ser559→His, Ser641→Asp and Ser1808→Glu;
(iii) Lys1773→Glu, optionally Glu1862→Asp and Gly235→Glu; and/or (iv) Ser199→Glu, Tyr204→His, Lys286→Asp and Gln590→Glu;
wherein the amino acid numbering refers to the amino acid sequence as shown in SEQ ID NO:4.

7. A modified homologue of human ceruloplasmin, wherein the modified homologue comprises at least one metal binding site (i) that is not present in the wild type-form of the homologue of human ceruloplasmin and (ii) that corresponds to a metal binding site or to an incomplete metal binding site present in human ceruloplasmin.

8. A modified homologue according to claim 7, wherein the homologue of human ceruloplasmin is human blood coagulation Factor V or human blood coagulation Factor VIII.

9. A modified homologue according to claim 7 or 8, wherein the metal binding site or incomplete metal binding site in human ceruloplasmin is formed by one of the following groups of amino acids:
(i) His637, Cys680, His 685 and Met 690;
(ii) Glu597, His602, Asp684 and Glu971;
(iii) Glu935, His940, Asp1025 and Glu272; or (iv) Glu236, Tyr241, Asn323 and Glu633;
wherein the amino acid numbering refers to the amino acid sequence as shown in SEQ ID NO:5.

10. A modified homologue according to any one of claims 7 to 9, wherein the homologue of human ceruloplasmin is human blood coagulation Factor VIII and has at least one of the following groups (i) to (iv) of amino acids at the indicated positions:
(i) His649, Cys692, His697 and Met702;
(ii) Glu610, His615, Asp696 and Glu1950;
(iii) Glu1914, His1919, optionally Asp2004 and Glu263; and/or (iv) Glu227, His232, Asp314 and Glu645;
wherein the amino acid numbering refers to the amino acid sequence as shown in SEQ ID NO:2.

11. A modified homologue according to any one of claims 7 to 9, wherein the homologue of human ceruloplasmin is human blood coagulation Factor V and has at least one of the following groups (i) to (iv) of amino acids at the indicated positions:
(i) His594, Cys637, His642, and Met647;
(ii) Glu554, His559, Asp641 and Glu1808;
(iii) Glu1773, His1778, optionally Asp1862 and Glu235; and/or (iv) Glu199, His204, Asp286 and Glu590;
wherein the amino acid numbering refers to the amino acid sequence as shown in SEQ ID NO:4.

12. A modified homologue according to any one of claims 7 to 11, wherein the modified homologue has an increased stability compared to the non-modified form of the homologue of human ceruloplasmin.

13. A polynucleotide encoding a modified homologue of human ceruloplasmin according to any one of claims 7 to 12.

14. A plasmid or vector comprising a nucleic acid according to claim 13.

15. A plasmid or vector according to claim 14 which is an expression vector.

16. A vector according to claim 14 which is a transfer vector for use in human gene therapy.

17. A host cell comprising a polynucleotide according to claim 13 or a plasmid or vector according to any one of claims 14 to 16.

18. A method of producing a modified homologue of human ceruloplasmin, comprising:
(c) culturing host cells according to claim 17 under conditions such that the modified homologue of human ceruloplasmin is expressed; and
(d) optionally recovering the modified homologue of human ceruloplasmin from the host cells or from the culture medium.

19. A pharmaceutical composition comprising a modified homologue of human ceruloplasmin according to any one of claims 7 to 12, a polynucleotide according to claim 13, or a plasmid or vector according to any one of claims 14 to 16.

20. The use of a modified homologue of human ceruloplasmin according to any one of claims 7 to 12, of a polynucleotide according to claim 13, of a plasmid or vector according to any one of claims 14 to 16, or of a host cell according to claim 17 for the manufacture of a medicament for the treatment or prevention of a blood coagulation disorder.

21. The use according to claim 19, wherein the blood coagulation disorder is hemophilia A.

22. The use according to claim 20 or 21, wherein the treatment comprises human gene therapy.
